# FASCICULE DE BREVET EUROPEEN

(11) **EP 2 066 807 B1**
(45) Date de publication et mention de la délivrance du brevet: **16.12.2009**
(21) Numéro de dépôt: 07823868.0
(22) Date de dépôt: 27.09.2007
(51) Int. Cl.: C12Q 1/68

(54) **PROCEDE DE DIAGNOSTIC IN VITRO DU CANCER BRONCHO-PULMONAIRE PAR DETECTION DES TRANSCRITS MAJORITAIRES ALTERNATIFS DU GENE KLK8 CODANT LA KALLICREINE 8 ET SON UTILISATION POUR LE PRONOSTIC DE SURVIE**
VERFAHREN ZUR IN-VITRO-DIAGNOSE VON LUNGENKARZINOMEN DURCH NACHWEIS GRÖSSERER ALTERNATIVER TRANSKRIPTE DES KLK8-GENS ZUR KODIERUNG VON KALLICREIN 8 UND SEINE VERWENDUNG ZUR PROGNOSTIZIERUNG VON ÜBERLEBENSCHANCEN
METHOD FOR THE IN VITRO DIAGNOSIS OF BRONCHOPULMONARY CARCINOMA BY DETECTING MAJOR ALTERNATIVE TRANSCRIPTS OF THE KLK8 GENE ENCODING KALLICREIN 8 AND USE THEREOF FOR PROGNOSTICATING SURVIVAL

(30) Priorité: 28.09.2006 FR 0653983
(43) Date de publication de la demande: 10.06.2009
(73) Titulaire: bioMérieux S.A., 69280 Marcy l'Etoile (FR); Universite Francois Rabelais de Tours, 37041 Tours Cedex 1 (FR)
(72) Inventeur: AINCIBURU, Mireille, 34160 Sussargues (FR); COURTY, Yves, 37000 Tours (FR); JOLIVET-REYNAUD, Colette, 69720 Saint Bonnet De Mure (FR); PLANQUE, Chris, 44380 Pornichet (FR)
(74) Mandataire: Bitaud, Valérie Marie-Odile
(86) Numéro de dépôt international: PCT/FR2007/052023
(87) Numéro de publication internationale: WO 2008/037930

(56) Documents cités:
- US-A1- 2006 205 054
- PLANQUE CHRIS ET AL: "Expression of the human kallikrein genes 10 (KLK10) and 11 (KLK11) in cancerous and non-cancerous lung tissues" BIOLOGICAL CHEMISTRY, vol. 387, no. 6, juin 2006 (2006-06), pages 783-788, XP002422266 ISSN: 1431-6730
- SHER YUH-PYNG ET AL: "Human kallikrein 8 protease confers a favorable clinical outcome in non-small cell lung cancer by suppressing tumor cell invasiveness" CANCER RESEARCH, vol. 66, no. 24, 15 décembre 2006 (2006-12-15), pages 11763-11770, XP002422267 ISSN: 0008-5472
- SINGH ET AL: "Expression of kallikrein-related peptidases (KRP/hK5, 7, 6, 8) in subtypes of human lung carcinoma" INTERNATIONAL IMMUNOPHARMACOLOGY, ELSEVIER, AMSTERDAM, NL, vol. 8, no. 2, 6 septembre 2007 (2007-09-06), pages 300-306, XP022411086 ISSN: 1567-5769
- MAGKLARA A ET AL: "The human KLK8 (neuropsin/ovasin) gene: identification of two novel splice variants and its prognostic value in ovarian cancer" CLINICAL CANCER RESEARCH, THE AMERICAN ASSOCIATION FOR CANCER RESEARCH, US, vol. 7, no. 4, avril 2001 (2001-04), pages 806-811, XP002244127 ISSN: 1078-0432
- SHIGEMASA K ET AL: "HUMAN KALLIKREIN 8 (HK8/TADG-14) EXPRESSION IS ASSOCIATED WITH AN EARLY CLINICAL STAGE AND FAVORABLE PROGNOSIS IN OVARIAN CANCER" ONCOLOGY REPORTS, NATIONAL HELLENIC RESEARCH FOUNDATION, ATHENS, GR, vol. 4, no. 6, juin 2004 (2004-06), pages 1153-1159, XP009040212 ISSN: 1021-335X
- PLANQUE C ET AL: "KLK5 and KLK7, two members of the human tissue kallikrein family, are differentially expressed in lung cancer" BIOCHEMICAL AND BIOPHYSICAL RESEARCH COMMUNICATIONS, ACADEMIC PRESS INC. ORLANDO, FL, US, vol. 329, no. 4, 22 avril 2005 (2005-04-22), pages 1260-1266, XP004784868 ISSN: 0006-291X cité dans la demande
- YOUSEF G M ET AL: "THE NEW HUMAN TISSUE KALLIKREIN GENE FAMILY: STRUCTURE, FUNCTION AND ASSOCIATION TO DISEASE" ENDOCRINE REVIEWS, BALTIMORE, MD, US, vol. 22, no. 2, avril 2001 (2001-04), pages 184-204, XP001104999

### SEQUENCE LISTING

<110> bioMérieux
   Université François Rabelais
<120> Procédé de diagnostic in vitro du cancer broncho-pulmonaire par détection des transcrits majoritaires alternatifs du gène KLK8 codant la kallicréine 8 et son utilisation pour le pronostic de survie
<130> Trans KLK8
<160> 32
<170> PatentIn version 3.3
<210> 1
   <211> 30
   <212> DNA
   <213> Artificial sequence
<220>
   <223> amorce d'amplification
<400> 1
   tggagggcgg ccgcatggga cgcccccgac 30
<210> 2
   <211> 27
   <212> DNA
   <213> Artificial sequence
<220>
   <223> amorce d'amplification
<400> 2
   tcctagatat cgcccttgct gcctatg 27
<210> 3
   <211> 806
   <212> DNA
   <213> Artificial sequence
<220>
   <223> Transcrit NT1 de la kallicréine 8
<400> 3
   tggaagggcg gccgcatggg acgcccccga cctcgtgcgg ccaagacgtg gatgttcctg     60
   ctcttgctgg ggggagcctg ggcaggacac tccagggcac aggaggacaa ggtgctgggg    120
   ggtcatgagt gccaacccca ttcgcagcct tggcaggcgg ccttgttcca gggccagcaa    180
   ctactctgtg gcggtgtcct tgtaggtggc aactgggtcc ttacagctgc ccactgtaaa    240
   aaaccgaaat acacagtacg cctgggagac cacagcctac agaataaaga tggcccagag    300
   caagaaatac ctgtggttca gtccatccca cacccctgct acaacagcag cgatgtggag    360
   gaccacaacc atgatctgat gcttcttcaa ctgcgtgacc aggcatccct ggggtccaaa    420
   gtgaagccca tcagcctggc agatcattgc acccagcctg gccagaagtg caccgtctca    480
   ggctggggca ctgtcaccag tccccgagag aattttcctg acactctcaa ctgtgcagaa    540
   gtaaaaatct ttccccagaa gaagtgtgag gatgcttacc cggggcagat cacagatggc    600
   atggtctgtg caggcagcag caaaggggct gacacgtgcc agggcgattc tggaggcccc    660
   ctggtgtgtg atggtgcact ccagggcatc acatcctggg gctcagaccc ctgtgggagg    720
   tccgacaaac ctggcgtcta taccaacatc tgccgctacc tggactggat caagaagatc    780
   ataggcagca agggcgatat ctagga                                         806
<210> 4
   <211> 940
   <212> DNA
   <213> Artificial sequence
<220>
   <223> Transcrit NT2 de la kallicréine 8
<400> 4
   tggaaggcgg ccgcatggga cgcccccgac ctcgtgcggc caagacgtgg atgttcctgc     60
   tcttgctggg gggagcctgg gcagcgtgtg gaagcctgga cctcctcact aagttgtatg    120
   cggagaactt gccgtgtgtc catttgaacc cacagtggcc ttcccagccc tcgcactgcc    180
   ccagagggtg gcgatccaac cctctccctc ctgctgcagg acactccagg gcacaggagg    240
   acaaggtgct ggggggtcat gagtgccaac cccattcgca gccttggcag gcggccttgt     300
   tccagggcca gcaactactc tgtggcggtg tccttgtagg tggcaactgg gtccttacag    360
   ctgcccactg taaaaaaccg aaatacacag tacgcctggg agaccacagc ctacagaata    420
   aagatggccc agagcaagaa atacctgtgg ttcagtccat cccacacccc tgctacaaca    480
   gcagcgatgt ggaggaccac aaccatgatc tgatgcttct tcaactgcgt gaccaggcat    540
   ccctggggtc caaagtgaag cccatcagcc tggcagatca ttgcacccag cctggccaga    600
   agtgcaccgt ctcaggctgg ggcactgtca ccagtccccg agagaatttt cctgacactc    660
   tcaactgtgc agaagtaaaa atctttcccc agaagaagtg tgaggatgct tacccggggc    720
   agatcacaga tggcatggtc tgtgcaggca gcagcaaagg ggctgacacg tgccagggcg    780
   attctggagg ccccctggtg tgtgatggtg cactccaggg catcacatcc tggggctcag   840
   acccctgtgg gaggtccgac aaacctggcg tctataccaa catctgccgc tacctggact    900
   ggatcaagaa gatcataggc agcaagggcg atatctagga                          940
<210> 5
   <211> 383
   <212> DNA
   <213> Artificial sequence
<220>
   <223> Transcrit NT3 de la kallicréine 8
<400> 5
   tggaaggcgg ccgcatggga cgcccccgac ctcgtgcggc caagacgtgg atgttcctgc     60
   tcttgctggg gggagcctgg gcagagaatt ttcctgacac tctcaactgt gcagaagtaa    120
   aaatctttcc ccagaagaag tgtgaggatg cttacccggg gcagatcaca gatggcatgg    180
   tctgtgcagg cagcagcaaa ggggctgaca cgtgccaggg cgattctgga ggccccctgg   240
   tgtgtgatgg tgcactccag ggcatcacat cctggggctc agacccctgt gggaggtccg    300
   acaaacctgg cgtctatacc aacatctgcc gctacctgga ctggatcaag aagatcatag    360
   gcagcaaggg cgatatctag gga                                            383
<210> 6
   <211> 248
   <212> DNA
   <213> Artificial sequence
<220>
   <223> Transcrit NT4 de la kallicréine 8
<400> 6
   tggaaggcgg ccgcatggga cgcccccgac ctcgtgcggc caagacgtgg atgttcctgc    60
   tcttgctggg gggagcctgg gcagggcgat tctggaggcc ccctggtgtg tgatggtgca   120
   ctccagggca tcacatcctg gggctcagac ccctgtggga ggtccgacaa acctggcgtc   180
   tataccaaca tctgccgcta cctggactgg atcaagaaga tcataggcag caagggcgat   240
   atctagga                                                            248
<210> 7
   <211> 645
   <212> DNA
   <213> Artificial sequence
<220>
   <223> Transcrit NT5 de la kallicréine 8
<400> 7
   tggaaggcgg ccgcatggga cgcccccgac ctcgtgcggc caagacgtgg atgttcctgc    60
   tcttgctggg gggagcctgg gcaggaaata cacagtacgc ctgggagacc acagcctaca   120
   gaataaagat ggcccagagc aagaaatacc tgtggttcag tccatcccac acccctgcta   180
   caacagcagc gatgtggagg accacaacca tgatctgatg cttcttcaac tgcgtgacca   240
   ggcatccctg gggtccaaag tgaagcccat cagcctggca gatcattgca cccagcctgg   300
   ccagaagtgc accgtctcag gctggggcac tgtcaccagt ccccgagaga attttcctga   360
   cactctcaac tgtgcagaag taaaaatctt tccccagaag aagtgtgagg atgcttaccc   420
   ggggcagatc acagatggca tggtctgtgc aggcaacagc aaaggggctg acacgtgcca   480
   gggcgattct ggaggccccc tggtgtgtga tggtgcactc cagggcatca catcctgggg   540
   ctcagacccc tgtgggaggt ccgacaaacc tggcgtctat accaacatct gccgctacct   600
   ggactggatc aagaagatca taggcagcaa gggcgatatc tagga                   645
<210> 8
   <211> 542
   <212> DNA
   <213> Artificial sequence
<220>
   <223> Transcrit NT6 de la kallicréine 8
<400> 8
   tggaaggcgg ccgcatggga cgcccccgac ctcgtgcggc ctagacgtgg atgttcctgc     60
   tcttgctggg gggagcctgg gcaggacact ccagggcaca ggaggacaag gtgctggggg    120
   gtcatgagtg ccaaccccat tcgcagcctt ggcaggtggc cttgttccag ggccagcaac    180
   tactctgtgg cggtgtcctt gtaggtggca actgggtcct tacagctgcc cactgtaaaa    240
   aaccagaatt ttcctgacac tctcaactgt gcagaagtaa aaatctttcc ccagaagaag    300
   tgtgaggatg cttacccggg gcagatcaca gatggcatgg tctgtgcagg cagcagcaaa    360
   ggggctgaca cgtgccaggg cgattctgga ggccccctgg tgtgtgatgg tgcactccag    420
   ggcatcacat cctggggctc agacccctgt gggaggtccg acaaacctgg cgtctatacc    480
   aacatctgcc gctacctgga ctggatcaag aagatcatag gcagcaaggg cgatatctag    540
   ga                                                                   542
<210> 9
   <211> 18
   <212> DNA
   <213> Artificial sequence
<220>
   <223> amorce d'amplification
<400> 9
   ggagcctggg cagagaat 18
<210> 10
   <211> 16
   <212> DNA
   <213> Artificial sequence
<220>
   <223> Amorce d'amplification
<400> 10
   tgggcagggc gattct 16
<210> 11
   <211> 24
   <212> DNA
   <213> Artificial sequence
<220>
   <223> amorce d'amplification
<400> 11
   ctaccacatc caaggaaggc agca 24
<210> 12
   <211> 24
   <212> DNA
   <213> Artificial sequence
<220>
   <223> amorce d'amplification
<400> 12
   gctatcaatc tgtcaatcct gtcc 24
<210> 13
   <211> 16
   <212> DNA
   <213> Artificial sequence
<220>
   <223> amorce d'amplification
<400> 13
   cctccagaat cgccct 16
<210> 14
   <211> 18
   <212> DNA
   <213> Artificial sequence
<220>
   <223> amorce d'amplification
<400> 14
   cagtccaggt agcggcag 18
<210> 15
   <211> 23
   <212> DNA
   <213> Artificial sequence
<220>
   <223> amorce d'amplification
<400> 15
   cgcggttcta ttttgttggt ttt 23
<210> 16
   <211> 20
   <212> DNA
   <213> Artificial sequence
<220>
   <223> amorce d'amplification
<400> 16
   ttcgctctgg tccgtcttgc 20
<210> 17
   <211> 20
   <212> DNA
   <213> Artificial sequence
<220>
   <223> amorce d'amplification
<400> 17
   gccactactc cctgtcacca 20
<210> 18
   <211> 20
   <212> DNA
   <213> Artificial sequence
<220>
   <223> amorce d'amplification
<400> 18
   gcatcctcgc accttttctg 20
<210> 19
   <211> 17
   <212> DNA
   <213> Artificial sequence
<220>
   <223> amorce d'amplification
<400> 19
   tgatggtggt gctgagt 17
<210> 20
   <211> 19
   <212> DNA
   <213> Artificial sequence
<220>
   <223> amorce d'amplification
<400> 20
   acagtggatg gataaggac 19
<210> 21
   <211> 18
   <212> DNA
   <213> Artificial sequence
<220>
   <223> amorce d'amplification
<400> 21
   gagcccagat gtgacctt 18
<210> 22
   <211> 19
   <212> DNA
   <213> Artificial sequence
<220>
   <223> amorce d'amplification
<400> 22
   tccttgtaaa ccttcgtgc 19
<210> 23
   <211> 17
   <212> DNA
   <213> Artificial sequence
<220>
   <223> amorce d'amplification
<400> 23
   ggaccccgaa gcctatg 17
<210> 24
   <211> 17
   <212> DNA
   <213> Artificial sequence
<220>
   <223> amorce d'amplification
<400> 24
   cctgagccct ggtggta 17
<210> 25
   <211> 20
   <212> DNA
   <213> Artificial sequence
<220>
   <223> amorce d'amplification
<400> 25
   caggatcatc aaggggttcg 20
<210> 26
   <211> 20
   <212> DNA
   <213> Artificial sequence
<220>
   <223> amorce d'amplification
<400> 26
   cattgcggtg gtctttgttg 20
<210> 27
   <211> 19
   <212> DNA
   <213> Artificial sequence
<220>
   <223> amorce d'amplification
<400> 27
   gtgccaacat ccaacttcg 19
<210> 28
   <211> 19
   <212> DNA
   <213> Artificial sequence
<220>
   <223> amorce d'amplification
<400> 28
   ccctcacagg agtctttgc 19
<210> 29
   <211> 19
   <212> DNA
   <213> Artificial sequence
<220>
   <223> amorce d'amplification
<400> 29
   tgggtcatca ctgctgctc 19
<210> 30
   <211> 19
   <212> DNA
   <213> Artificial sequence
<220>
   <223> amorce d'amplification
<400> 30
   ctcctcaggt tgtgcttgc 19
<210> 31
   <211> 20
   <212> DNA
   <213> Artificial sequence
<220>
   <223> amorce d'amplification
<400> 31
   ccagaagaag tgtgaggatg 20
<210> 32
   <211> 19
   <212> DNA
   <213> Artificial sequence
<220>
   <223> amorce d'amplification
<400> 32
   ggtatagacg ccaggtttg 19

## Description

La présente invention concerne le domaine de la cancérologie. Plus particulièrement, la présente invention a pour objet un procédé de diagnostic *in vitro* du cancer broncho-pulmonaire primitif chez un patient humain par détermination de la présence au-delà d'un seuil prédéterminé, d'un transcrit majoritaire du gène *KLK8* de la kallicréine 8 dans un échantillon biologique issu de ce patient.

Le cancer broncho-pulmonaire primitif est la première cause de décès par cancer chez l'homme et ce, dans tous les pays développés. Des données récentes montrent une nette augmentation de l'incidence chez les femmes. On estime le nombre de nouveaux cas par an à 25 000 en France et à plus de 160 000 aux Etats-Unis, entraînant le décès annuel d'environ 22 000 individus en France et 155 000 aux Etats-Unis. Au niveau mondial, le cancer broncho-pulmonaire serait responsable d'environ 900 000 décès chaque année, ce qui correspondrait environ à 18% des décès par cancer. L'étiologie principale du cancer broncho-pulmonaire est le tabagisme. Environ 90% des cancers bronchiques chez l'homme et environ 50% chez la femme sont attribuables au tabac. D'autres facteurs environnementaux ou professionnels peuvent également être reconnus dans la carcinogénèse bronchique.

L'Organisation Mondiale de la Santé (OMS) distingue les cancers bronchiques à petites cellules (CBPC), qui représentent environ 20% des cas, et les cancers bronchiques non à petites cellules (CBNPC) qui regroupent entre autres les carcinomes épidermoïdes, les adénocarcinomes, et les carcinomes à grandes cellules, et qui représentent environ 80% des cas. Les carcinomes épidermoïdes et les adénocarcinomes sont les cancers les plus répandus.

A l'heure actuelle, le diagnostic du cancer broncho-pulmonaire se fait essentiellement par radiographie pulmonaire et scanner thoracique. Une endoscopie bronchique permettant la réalisation de biopsies confirme ensuite le diagnostic. Malheureusement, les symptômes évocateurs sont malheureusement tardifs et peu spécifiques, et le diagnostic intervient tardivement, réduisant ainsi grandement l'efficacité et la faisabilité des traitements existants. De plus, ce type de diagnostic nécessite un matériel performant et du personnel qualifié ce qui est coûteux.

Différentes méthodes de traitement sont actuellement disponibles: la chirurgie, la chimiothérapie, la radiothérapie. Ces traitements peuvent être réalisés, soit de manière isolée, soit de manière séquentielle ou combinée.

Le taux de survie du cancer du poumon est très dépendant du degré de dissémination de la tumeur au moment du diagnostic. Le taux global de survie à 5 ans est de l'ordre de 15%. Ce taux masque cependant des disparités importantes. Le taux de survie des patients ayant un cancer métastasé à distance lors du diagnostic est inférieur à 5 % tandis que les patients dont le cancer «non à petites cellules » (CBNPC) est localisé au moment de sa découverte présentent des taux de survie proches de 50%¹. Ces derniers sont essentiellement traités par résection chirurgicale de la tumeur, approche qui pour l'instant représente la seule solution curative pour ce type de cancer. Toutefois, moins d'un patient sur 3 peut faire l'objet d'un tel traitement et un patient sur 2 traités par chirurgie décède dans les mois suivants l'opération, suite à une récidive tumorale. Les progrès récents dans le domaine de la chimiothérapie moderne du CBNPC permettent d'envisager des traitements adjuvants ou néo-adjuvants améliorant l'espérance de vie des patients opérés². De tels traitements ne sont cependant pas anodins avec un taux de mortalité associé non négligeable. Dans ce contexte, il est important de pouvoir identifier les patients opérables présentant un haut risque de décès par récidive, afin de faciliter la décision d'appliquer ou non une chimiothérapie néo-adjuvante ou adjuvante.

Des marqueurs qui permettent de distinguer les cellules tumorales des cellules saines sont recherchés et étudiés depuis des années pour tous les cancers et notamment du cancer broncho-pulmonaire. Ils permettraient de diagnostiquer précocement la maladie, d'en établir le pronostic et la sensibilité au traitement, et d'en surveiller l'évolution. Dans les dernières années, plus de 100 candidats ont été suggérés comme marqueurs moléculaires de diagnostic du cancer broncho-pulmonaire. Les études ont ainsi envisagé le rôle diagnostic de proto-oncogènes, de facteurs impliqués dans le cycle cellulaire, l'apoptose ou l'angiogenèse. Cependant, peu de corrélations entre les résultats obtenus par différentes techniques et de validations entre différentes cohortes de patients ont pu être obtenues selon la technique employée (immunohistochimie, dosage immunologique, puces à ADN utilisant différents algorithmes) et la grande diversité des tumeurs (type histologique, stade, degré de différenciation).

D'autres marqueurs, appartenant à une sous-famille de protéases à sérine, les kallicréines, dont on en dénombre 15, ont également été testés. Ainsi, dans une étude par puces à ADN, le gène *hKLK11* a été identifié comme marqueur des adénocarcinomes endocrines de type C2³. Une étude similaire a montré que les gènes *hKLK5* et *hKLK10* sont surexprimés dans les carcinomes épidermoïdes⁴. De même, il a été montré que les gènes *hKLK5* et *hKLK7*, codant respectivement les protéines hK5 et hK7, étaient surexprimés dans les tissus tumoraux de carcinomes épidermoïdes, alors qu'une sous-expression du gène *hKLK7* dans les tissus tumoraux est le plus souvent observée chez les patients présentant un adénocarcinome⁵. Cependant, aucun lien entre l'expression différentielle de ces gènes *hKLK* et un pronostic vital pour les patients atteints d'un cancer broncho-pulmonaire n'a pu être établi.

Les gènes des 15 kallicréines présentent des caractéristiques communes, dont la présence de plusieurs transcrits pour le même gène. Les transcrits de ces gènes ont également été étudiés en tant que marqueurs. Ainsi, il a été indiqué que trois transcrits alternatifs des gènes *hKLK4*⁶, *hKLK5*⁷, un transcrit du gène *hKLK7*⁷ étaient sur-exprimés dans les tumeurs et/ou dans des lignées cellulaires ovariennes comparativement au tissu non cancéreux.

Il est connu que le profil d'expression du gène *hKLK8* donne lieu à au moins 4 transcrits différents, appelés NT1 à NT4. Le transcript NT1 ou « neuropsine type 1», identifié par Yoshida S. et al⁸, code une préproenzyme de 260 acides aminés contenant un peptide signal de sécrétion de 28 acides aminés et un très court prosegment de 4 résidus devant être clivé pour libérer la forme active de la kallicréine 8. NT1 est considérée comme la forme régulière d'expression du gène. Le transcript NT2 ou « neuropsine-T2 », identifié par Mitsui S. et al⁹, se différencie de la forme NT1 par l'insertion d'une séquence codant 45 acides aminés supplémentaires dans la région carboxy-terminale du peptide signal. Les transcrits NT3 et NT4 ont été identifiés par Magklara A. et al¹⁰ et codent des protéines contenant respectivement 119 et 32 résidus. La forme protéique prédite à partir de NT3 ne possède qu'une partie du peptide signal de kallicréine 8 et ne conserve pas la zone de clivage de celui-ci. La protéine prédite à partir de NT4 est constituée des 23 premiers résidus du peptide signal de la kallicréine 8 et de 9 résidus supplémentaires sans identité avec la kallicréine 8. Magklara A. et al¹⁰ ont montré que, bien que la forme régulière d'expression du gène *KLK8*, NT1, ait une valeur pronostique dans le cadre du cancer de l'ovaire, les formels NT3 et NT4 n'ont aucune valeur.

Les Demanderesses ont maintenant mis en évidence de façon surprenante que les transcrits majoritaires alternatifs du gène *KLK8* codant la kallicréine 8, choisis parmi NT3 et NT4, en un taux élevé, étaient un bon marqueur de diagnostic, et notamment de pronostic défavorable, dans le cadre des cancers broncho-pulmonaires, et en particulier des cancers bronchiques non à petites cellules.

Ainsi, la présente invention a pour premier objet un procédé de diagnostic *in vitro* du cancer broncho-pulmonaire, notamment du cancer bronchique non à petites cellules, **caractérisé en ce qu**'il comprend ou consiste en l'étape de détection, dans un échantillon biologique issu d'un patient suspecté d'être atteint dudit cancer broncho-pulmonaire, d'au moins un des transcrits majoritaires alternatifs du gène *KLK8* de la kallicréine 8.

Le procédé de l'invention permet donc d'établir un diagnostic dans le cadre du cancer broncho-pulmonaire par un test simple consistant à détecter les transcrits majoritaires alternatifs du gène *KLK8*, notamment en un taux élevé.

Par transcrit alternatif du gène *KLK8*, on entend les produits de transcription du gène *KLK8*. De tels transcrits sont tels que décrits précédemment et sont appelés, notamment, NT1, NT2, NT3 et NT4. Comme on le verra plus loin, les Demanderesses ont découvert deux nouveaux transcrits dénommés NT5 et NT6.

Par transcrit majoritaire, on entend les transcrits produits en majorité à partir du gène d'expression de la la kallicréine 8. Selon un mode de réalisation, les transcrits majoritaires alternatifs du gène *KLK8* sont choisis parmi les transcrits NT3 et NT4.

Par taux élevé de transcrit majoritaires, on entend un taux supérieur à une valeur-seuil déterminée.

On sait que, de façon générale, les résultats des tests de détection d'analytes dépendent en grande partie des caractéristiques des partenaires de liaison utilisés. Ainsi, par exemple, dans le cas de la détection d'ARN par hybridation avec des sondes nucléotidiques, les résultats dépendent en particulier des caractéristiques de taille, de composition et de pourcentage de complémentarité des sondes, et que ces caractéristiques influent sur les valeurs mesurées avec ces sondes. On conçoit donc qu'il n'est pas possible de donner des valeurs-seuils précises et que des valeurs-seuils adaptées à chaque partenaire de liaison utilisé peuvent être déterminées dans chaque cas par de simples expériences de routine.

Il faut bien comprendre que l'on appelle ici valeur-seuil soit une valeur discrète, soit un intervalle de valeurs correspondant à une zone d'indétermination. Bien évidemment, lorsque la valeur mesurée est incluse dans l'intervalle d'indétermination, ou est très proche de la valeur-seuil dans le cas d'une valeur discrète, on ne peut pas conclure définitivement et il convient de conduire des investigations supplémentaires.

Les échantillons biologiques dans lesquels le procédé de l'invention est mis en oeuvre sont tout échantillon biologique susceptible de contenir des transcrits majoritaires alternatifs du gène *KLK8*. A titre d'exemple de tels échantillons, on peut citer les échantillons solides tels que le tissu provenant de la biopsie de la tumeur, de ganglions lymphatiques, des métastases du patient, les liquides biologiques tels que le sang, le sérum, le plasma et les expectorations, et les cellules purifiées à partir de ces échantillons solides ou liquides.

On entend par détection de transcrit, en particulier du transcrit majoritaire alternatif, soit la détection directe du transcrit, soit la détection indirecte du transcrit, ou tout autre procédé de détermination de la présence d'un ARN dans un échantillon, connu de l'homme du métier.

Par détection directe du transcrit, en particulier du transcrit majoritaire alternatif, on entend la mise en évidence dudit transcrit lui-même dans l'échantillon biologique.

La détection directe du transcrit majoritaire alternatif dans l'échantillon biologique peut être mise en oeuvre par tout moyen connu de l'homme du métier, comme par exemple par hybridation avec un partenaire de liaison spécifique du transcrit majoritaire alternatif, le cas échéant après amplification par la technique PCR ou NASBA.

Par hybridation, on entend le processus au cours duquel, dans des conditions appropriées, deux fragments nucléotidiques se lient avec des liaisons hydrogènes stables et spécifiques pour former un complexe double brin. Ces liaisons hydrogènes se forment entre les bases complémentaires Adénine (A) et thymine (T) (ou uracile (U)) (on parle de liaison A-T) ou entre les bases complémentaires Guanine (G) et cytosine (C) (on parte de liaison G-C). L'hybridation de deux fragments nucléotidiques peut être totale (on parle alors de fragments nucléotidiques ou de séquences complémentaires), c'est-à-dire que le complexe double brin obtenu lors de cette hybridation comprend uniquement des liaisons A-T et des liaisons C-G. Cette hybridation peut être partielle (on parle alors de fragments nucléotidiques ou de séquences suffisamment complémentaires), c'est-à-dire que le complexe double brin obtenu comprend des liaisons A-T et des liaisons C-G permettant de former le complexe double brin, mais également des bases non liées à une base complémentaire. L'hybridation entre deux fragments nucléotidiques dépend des conditions opératoires qui sont utilisées, et notamment de la stringence. La stringence est définie notamment en fonction de la composition en bases des deux fragments nucléotidiques, ainsi que par le degré de mésappariement entre deux fragments nucléotidiques. La stringence peut également être fonction des paramètres de la réaction, tels que la concentration et le type d'espèces ioniques présentes dans la solution d'hybridation, la nature et la concentration d'agents dénaturants et/ou la température d'hybridation. Toutes ces données sont bien connues et les conditions appropriées peuvent être déterminées par l'homme du métier. En général, selon la longueur des fragments nucléotidiques que l'on souhaite hybrider, la température d'hybridation est comprise entre environ 20 et 70°C, en particulier entre 35 et 65°C dans une solution saline à une concentration d'environ 0,5 à 1 M. Une séquence, ou fragment nucléotidique, ou oligonucléotide, ou polynucléotide, est un enchaînement de motifs nucléotidiques assemblés entre eux par des liaisons ester phosphorique, caractérisé par la séquence informationnelle des acides nucléiques naturels, susceptibles de s'hybrider à un fragment nucléotidique, l'enchaînement pouvant contenir des monomères de structures différentes et être obtenu à partir d'une molécule d'acide nucléique naturelle et/ou par recombinaison génétique et/ou par synthèse chimique. Un motif est dérivé d'un monomère qui peut être un nucléotide naturel d'acide nucléique dont les éléments constitutifs sont un sucre, un groupement phosphate et une base azotée ; dans l'ADN le sucre est le désoxy-2-ribose, dans l'ARN le sucre est le ribose ; selon qu'il s'agisse de l'ADN ou l'ARN, la base azotée est choisie parmi l'adénine, la guanine, l'uracile, la cytosine, la thymine ; ou bien le monomère est un nucléotide modifié dans l'un au moins des trois éléments constitutifs ; à titre d'exemple, la modification peut intervenir soit au niveau des bases, avec des bases modifiées telles que l'inosine, la méthyl-5désoxycytidine, la désoxyuridine, la diméthylamino-5désoxyuridine, la diamino-2,6-purine, la bromo-5désoxyuridine ou toute autre base modifiée capable d'hybridation, soit au niveau du sucre, par exemple le remplacement d'au moins un désoxyribose par un polyamide¹¹, soit encore au niveau du groupement phosphate, par exemple son remplacement par des esters notamment choisis parmi les diphosphates, alkyl- et aryl-phosphonates et phosphorothioates.

Les partenaires de liaison spécifiques du transcrit majoritaire alternatif sont tout partenaire susceptible de se lier au transcrit majoritaire alternatif. A titre d'exemple, on peut citer les sondes nucléiques, les amorces d'amplification, et toute autre molécule capable de se lier au transcrit majoritaire alternatif.

Par sonde d'hybridation, on entend un fragment nucléotidique comprenant de 5 à 100 motifs nucléiques, notamment de 10 à 35 motifs nucléiques, possédant une spécificité d'hybridation dans des conditions déterminées pour former un complexe d'hybridation avec le matériel spécifique d'un gène cible. Dans la présente invention, le matériel spécifique du gène cible peut être une séquence nucléotidique comprise dans un ARN messager issu du gène cible (on parle alors d'ARNm spécifique du gène cible), une séquence nucléotidique comprise dans un ADN complémentaire obtenu par transcription inverse dudit ARN messager (on parle alors d'ADNc spécifique du gène cible), ou encore une séquence nucléotidique comprise dans un ARN complémentaire obtenu par transcription dudit ADNc tel que décrit précédemment (on parlera alors d'ARNc spécifique du gène cible). La sonde d'hybridation peut comprendre un marqueur permettant sa détection.

On entend par amorce d'amplification, un fragment nucléotidique comprenant de 5 à 100 motifs nucléiques, préférentiellement de 15 à 30 motifs nucléiques permettant l'initiation d'une polymérisation enzymatique, telle que notamment une réaction d'amplification enzymatique. Par réaction d'amplification enzymatique, on entend un processus générant de multiples copies d'un fragment nucléotidique par l'action d'au moins une enzyme. De telles réactions d'amplification sont bien connues de l'homme du métier et on peut citer notamment les techniques suivantes :
- PCR (Polymerase Chain Reaction), telle que décrite dans les brevets US 4,683,195, US 4,683,202 et US 4,800,159,
- LCR (Ligase Chain Reaction), exposée par exemple dans la demande de brevet EP 0 201 184,
- RCR (Repair Chain Reaction), décrite dans la demande de brevet WO 90/01069,
- 3SR (Self Sustained Sequence Replication) avec la demande de brevet WO 90/06995,
- NASBA (Nucleic Acid Sequence-Based Amplification) avec la demande de brevet WO 91/02818, et
- TMA (Transcription Mediated Amplification) avec le brevet US 5,399,491.

Lorsque l'amplification enzymatique est une PCR, le réactif spécifique comprend au moins 2 amorces d'amplification, spécifiques d'un gène cible, et permettant l'amplification du matériel spécifique du gène cible. Le matériel spécifique du gène cible comprend alors préférentiellement un ADN complémentaire obtenu par transcription inverse d'ARN messager issu du gène cible (on parle alors d'ADNc spécifique du gène cible) ou un ARN complémentaire obtenu par transcription des ADNc spécifique d'un gène cible (on parle alors d'ARNc spécifique du gène cible). Lorsque l'amplification enzymatique est une PCR réalisée après une réaction de transcription reverse, on parle de RT-PCR.

Par détection, on entend soit une méthode physique, soit une méthode chimique avec un agent colorant intercalant tel que SYBR® Green I ou le bomure d'éthydium, soit une méthode de détection à l'aide d'un marqueur. De nombreuses méthodes de détection existent pour la détection des acides nucléiques¹²⁻¹³.

Par marqueur, on entend un traceur capable d'engendrer un signal que l'on peut détecter. Une liste non limitative de ces traceurs comprend les enzymes qui produisent un signal détectable par exemple par colorimétrie, fluorescence ou luminescence, comme la peroxydase de raifort, la phosphatase alcaline, la beta-galactosidase, la glucose-6-phosphate déshydrogénase; les chromophores comme les composés fluorescents, luminescents ou colorants ; les groupements à densité électronique détectables par microscopie électronique ou par leurs propriétés électriques comme la conductivité, par les méthodes d'ampérométrie ou de voltamétrie, ou par des mesures d'impédance ; les groupements détectables par des méthodes optiques comme la diffraction, la résonance plasmon de surface, la variation d'angle de contact ou par des méthodes physiques comme la spectroscopie de force atomique, l'effet tunnel, etc. ; les molécules radioactives comme ³²P, ³⁵S ou ¹²⁵I.

La sonde d'hybridation peut être une sonde dite de détection. Dans ce cas, h sonde dite de détection est marquée au moyen d'un marqueur tel que défini précédemment. Grâce à la présence de ce marqueur, on peut détecter la présence d'une réaction d'hybridation entre une sonde de détection donnée et le transcrit à détecter.

La sonde de détection peut être notamment une sonde de détection «molecular beacons »¹⁴. Ces "molecular beacons" deviennent fluorescentes lors de l'hybridation. Elles possèdent une structuré de type tige-boucle et contiennent un fluorophore et un groupe "quencher". La fixation de la séquence de boucle spécifique avec sa séquence complémentaire d'acide nucléique cible provoque un déroulement de la tige et l'émission d'un signal fluorescent lors de l'excitation à la longueur d'onde qui convient.

La sonde d'hybridation peut être également une sonde dite de capture. Dans ce cas, la sonde dite de capture est immobilisée ou immobilisable sur un support solide par tout moyen approprié, c'est-à-dire directement ou indirectement, par exemple par covalence ou adsorption. Comme support solide, on peut utiliser des matériaux de synthèse ou des matériaux naturels, éventuellement modifiés chimiquement, notamment les polysaccharides tels que les matériaux à base de cellulose, par exemple du papier, des dérivés de cellulose tels que l'acétate de cellulose et la nitrocellulose ou le dextrane, des polymères, des copolymères, notamment à base de monomères du type styrène, des fibres naturelles telles que le coton, et des fibres synthétiques telles que le nylon ; des matériaux minéraux tels que la silice, le quartz, des verres, des céramiques ; des latex ; des particules magnétiques ; des dérivés métalliques, des gels etc. Le support solide peut être sous la forme d'une plaque de microtitration, d'une membrane comme décrit dans la demande WO-A-94/12670, d'une particule. On peut également immobiliser sur le support plusieurs sondes de capture différentes, chacune étant spécifique d'un transcrit cible. En particulier, on peut utiliser comme support une biopuce sur laquelle peuvent être immobilisées un grand nombre de sondes. Par biopuce, on entend un support solide de dimension réduite où sont fixée une multitude de sondes de capture à des positions prédéterminées. Le concept de biopuce, ou puce à ADN, date du début des années 90. Il repose sur une technologie pluridisciplinaire intégrant la micro-électronique, la chimie des acides nucléiques, l'analyse d'images et l'informatique. Le principe de fonctionnement repose sur un fondement de la biologie moléculaire: le phénomène d'hybridation, c'est-à-dire l'appartement par complémentarité des bases de deux séquences d'ADN et/ou d'ARN. La méthode des biopuces repose sur l'emploi de sondes de capture fixées sur un support solide sur lesquelles on fait agir un échantillon de fragments nucléotidiques cibles marqués directement ou indirectement avec des fluorochromes. Les sondes de capture sont positionnées de manière spécifique sur le support ou puce et chaque hybridation donne une information particulière, en relation avec le fragment nucléotidique cible. Les informations obtenues sont cumulatives, et permettent par exemple de quantifier le niveau d'expression d'un gène/transcrit ou de plusieurs gènes/transcrits cibles. Après hybridation, le support ou puce est lavé(e), et les complexes ADNc ou ARNc maquées / sondes de capture sont révélés par un ligand de forte affinité lié par exemple à un marqueur de type fluorochrome. La fluorescence est lue par exemple par un scanner et l'analyse de la fluorescence est traitée par informatique. On peut citer à titre indicatif, les puces à ADN mises au point par la société Affymetrix ("Accessing Genetic Information with High-Density DNA arrys")^{15,16}, pour les diagnostics moléculaires. Dans cette technologie, les sondes de capture sont généralement de tailles réduites, autour de 25 nucléotides. D'autres exemples de biopuces sont donnés dans de nombreuses publications^{17, 18, 19, 20, 21} ou dans les brevets US-A-4,981,783, US-A-5,700,637, US-A-5,445,934, US-A-5,744,305 et US-A-5,807,522. La caractéristique principale du support solide doit être de conserver les caractéristiques d'hybridation des sondes de capture sur les fragments nucléotidiques cibles tout en générant un bruit de fond minimum pour la méthode de détection.

Pour l'immobilisation des sondes sur le support, on distingue trois grands types de fabrication.

Il y a, tout d'abord, une première technique qui consiste en un dépôt de sondes pré-synthétisées. La fixation des sondes se fait par transfert direct, au moyen de micropipettes, de micro-pointes ou par un dispositif de type jet d'encre. Cette technique permet la fixation de sondes de taille allant de quelques bases (5 à 10) jusqu'à des tailles relativement importantes de 60 bases (impression) à quelques centaines de bases (micro-déposition) :
. L'impression est une adaptation du procédé utilisé par les imprimantes à jet d'encre. Elle repose sur la propulsion de très petites sphères de fluide (volume <1 nl) et à un rythme pouvant attendre 4000 gouttes/secondes. L'impression n'implique aucun contact entre le système libérant le fluide et la surfacé sur laquelle il est déposé.
. La micro-déposition consiste à fixer des sondes longues de quelques dizaines à plusieurs centaines de bases à la surface d'une lame de verre. Ces sondes sont généralement extraites de bases de données et se présentent sous forme de produits amplifiés et purifiés. Cette technique permet de réaliser des puces dénommées microarrays portant environ dix mille spots, dit zones de reconnaissance, d'ADN sur une surface d'un peu moins de 4 cm². Il ne faut toutefois pas oublier l'emploi de membranes de Nylon, dites « macroarrays », qui portent des produits amplifiés, généralement par PCR, avec un diamètre de 0,5 à 1 mm et dont la densité maximale est de 25 spots/cm2. Cette technique très flexible est utilisée par de nombreux laboratoires, cette dernière technique est considérée comme faisant partie des biopuces. On peut toutefois déposer en fond de plaque de microtitration un certain volume d'échantillon dans chaque puits, comme c'est le cas dans les demandes de brevet WO-A-00/71750 et FR 00/14896, ou déposer au fond d'une même boîte de Pétri un certain nombre de gouttes séparées les unes des autres, selon une autre demande de brevet FR00/14691.

La deuxième technique de fixation des sondes sur le support ou puce est appelée la synthèse in situ. Cette technique aboutit à l'élaboration de sondes courtes directement à la surface de la puce. Elle repose sur la synthèse d'oligonucléotides in situ (voir notamment les demandes de brevet WO 89/10977 et WO 90/03382), et est fondée sur le procédé des synthétiseurs d'oligonucléotides. Elle consiste à déplacer une chambre de réactions, où se déroule la réaction d'élongation d'oligonucléotides, le long de la surface de verre.

Enfin, la troisième technique est appelée la photolithographie, qui est un procédé à l'origine des biopuces développées par Affymetrix. Il s'agit également d'une synthèse in situ. La photolithographie est dérivée des techniques des microprocesseurs. La surface de la puce est modifiée par la fixation de groupements chimiques photolabiles pouvant être activés par la lumière. Une fois illuminés, ces groupes sont susceptibles de réagir avec l'extrémité 3' d'un oligonucléotide. En protégeant cette surface par des masques de formes définies, on peut illuminer et donc activer sélectivement des zones de la puce où l'on souhaite fixer l'un ou l'autre des quatre nucléotides. L'utilisation successive de masques différents permet d'alterner des cycles de protection/réaction et donc de réaliser les sondes d'oligonucléotides sur des spots d'environ quelques dizaines de micromètre carré (µm²). Cette résolution permet de créer jusqu'à plusieurs centaines de milliers de spots sur une surface de quelques centimètre carré (cm²). La photolithographie présente des avantages : massivement parallèle, elle permet de créer une puce de N-mères en seulement 4 x N cycles. Toutes ces techniques sont bien entendues utilisables.

L'échantillon biologique utilisé pour la détection directe du transcrit majoritaire alternatif, susceptible de contenir du transcrit majoritaire alternatif en tant que tel, peut être constitué par du fluide biologique ou un tissu provenant de la biopsie de la tumeur des ganglions lymphatiques ou des métastases du patient considéré.

Pour détecter le transcrit de l'échantillon biologique, une étape d'extraction est généralement nécessaire. Celui-ci peut également être détecté sans extraction sur des coupes de tissu par des techniques d'hybridation *in situ*. L'extraction est mise en oeuvre par tous les protocoles d'extraction et de purification d'acides nucléiques bien connus de l'homme du métier. A titre indicatif, l'extraction d'acides nucléique peut être réalisée par :
■ une étape de lyse des cellules présentes dans l'échantillon biologique, afin de libérer les acides nucléiques contenus dans les cellules du patient. A titre d'exemple, on peut utiliser les méthodes de lyse telles que décrites dans les demandes de brevet:
   ○ WO 00/05338 sur la lyse mixte magnétique et mécanique,
   ○ WO 99/53304 sur la lyse électrique,
   ○ WO 99/15321 sur la lyse mécanique.
   L'homme du métier pourra utiliser d'autres méthodes de lyse bien connues, telles que les chocs thermiques ou osmotiques ou les lyses chimiques par des agents chaotropiques tels que les sels de guanidium (US 5,234,809).
■ une étape de purification, permettant la séparation entre les acides nucléiques et les autres constituants cellulaires relargués dans l'étape de lyse. Cette étape permet généralement de concentrer les acides nucléiques, et peut être adapté à la purification d'ARN. A titre d'exemple, on peut utiliser des particules magnétiques éventuellement revêtues d'oligonucléotides, par adsorption ou covalence (voir à ce sujet les brevets US 4,672,040 et US 5,750,338), et ainsi purifier les acides nucléiques qui se sont fixés sur ces particules magnétiques, par une étape de lavage. Cette étape de purification des acides nucléiques est particulièrement intéressante si l'on souhaite amplifier ultérieurement lesdits acides nucléiques. Un mode de réalisation particulièrement intéressant de ces particules magnétiques est décrit dans les demandes de brevet: WO-A-97/45202 et WO-A-99/35500. Un mode de réalisation particulièrement intéressant de ces particules magnétiques est décrit dans les demandes de brevet de silice soit sous forme de colonne, soit sous folle de particules inelies²² ou magnétiques (Merck: MagPre^{™} Silica, Promega: MagneSil^{™} Paramagnetic particles). D'autres méthodes très répandues reposent sur des résines échangeuses d'ions en colonne ou en format particulaire paramagnétique (Whatman: DEAE-Magarose)²³. Une autre méthode très pertinente est celle de l'adsorption sur support d'oxyde métallique (société Xtrana: matrice Xtra-Bind^{™}).

Lorsque l'on souhaite extraire spécifiquement les ARN d'un échantillon biologique, on peut notamment réaliser une extraction par du phénol, du chloroforme et de l'alcool pour éliminer les protéines et précipiter les ARN avec de l'éthanol 100%. Les ARN peuvent alors être culoté par centrifugation, lavé et remis en solution.

Le fluide biologique peut nécessiter un traitement particulier. Le transcrit majoritaire alternatif peut s'y trouver en solution ou contenu dans des cellules tumorales circulantes. Si la recherche du transcrit alternatif vise la fraction contenue dans les cellules tumorales, alors, le fluide biologique sera préalablement traité afin d'isoler les cellules tumorales circulantes contenues dans le dit fluide.

Par isoler les cellules tumorales circulantes, on entend obtenir une fraction cellulaire enrichie en cellules tumorales circulantes.

Le traitement du fluide pour isoler les cellules tumorales circulantes peut être effectué par tri cellulaire dans un cytomètre de flux, par enrichissement sur Ficoll, par enrichissement par billes magnétiques recouvertes d'anticorps spécifiques, ou par toute autre méthode d'enrichissement spécifique connue de l'homme du métier.

Les cellules tumorales circulantes peuvent être isolées grâce à une technique de séparation cellulaire sur Ficoll associée à une déplétion des cellules sanguines utilisant des anticorps anti-CD45 couplés à des billes magnétiques (Dynal Biotech ASA, Norvège).

La détection directe du transcrit majoritaire alternatif peut alors être effectuée directement à partir de cellules tumorales circulantes isolées du fluide biologique. Par exemple, les cellules tumorales circulantes déposées sur une lame par cytospin peuvent être mises en contact avec une sonde spécifique du transcrit majoritaire alternatif afin de réaliser une hybridation *in situ*.

Un exemple de méthode indirecte consiste à traduire *in vitro* les ARN extraits des échantillons en protéines, par exemple à l'aide de systèmes d'expression tels que *E. coli*, puis de détecter le produit de traduction spécifique de l'ARN alternatif par un test immunologique tel que les tests «sandwich », par exemple ELISA, ou de compétition. Ces méthodes sont largement connues de l'homme du métier et utilisent notamment des anticorps monoclonaux et/ou polyclonaux à titre de partenaire spécifique de liaison du peptide traduit de l'ARN.

Le procédé de l'invention peut être mis en oeuvre par les étapes consistant à :
i) déterminer la quantité de transcrit majoritaire alternatif dans l'échantillon biologique,
ii) comparer la quantité de transcrit majoritaire alternatif dans l'échantillon biologique à une valeur-seuil prédéterminée, choisie selon le type de dosage utilisé et représentative de la limite de détection de la pathologie et
iii) établir le diagnostic.

La quantification de la concentration en transcrit majoritaire alternatif peut être mise en oeuvre par tout procédé connu de l'homme du métier pour quantifier un marqueur dans un échantillon biologique, tel qu'en utilisant un test d'hybridation, comme décrit précédemment.

Egalement comme indiqué précédemment, on sait que, de façon générale, les résultats des tests de détection des acides nucléiques dépendent en grande partie des caractéristiques des partenaires de liaison utilisés, de sorte qu'il n'est pas possible de donner des valeurs-seuils précises et que des valeurs-seuils adaptées à chaque partenaire de liaison utilisé peuvent être déterminées dans chaque cas par de simples expériences de routine.

Le procédé de diagnostic de l'invention peut être amélioré en incluant également une étape supplémentaire de détection d'au moins un autre transcrit du gène *KLK8*, ce qui constitue un mode de réalisation particulier de l'invention.

Par autre transcrit du gène *KLK8*, on entend :
- les autres transcrits alternatifs du gène *KLK8*, appelés transcrits minoritaires, tels que ceux déjà connus comme NT2, NT3 et NT4, ainsi que de nouveaux transcrits découverts par les Demanderesses, appelés NT5 et NT6,
- le transcrit codant la kallicréine KLK8 également appelé NT1.

Les nouveaux transcrits NT5 et NT6, de séquence SEQ ID N°7 et SEQ ID N°8, respectivement, sont nouveaux et constituent un autre objet de l'invention.

Le procédé de diagnostic de l'invention incluant une étape supplémentaire de détection d'un autre transcrit alternatif du gène *KLK8* peut être mis en oeuvre les étapes consistant à :
i) déterminer la quantité de transcrit majoritaire alternatif du gène *KLK8* et d'autre transcrit, le cas échéant alternatif, du gène *KLK8* dans le même échantillon biologique, et
ii) comparer la quantité obtenue à une valeur-seuil prédéterminée, choisie selon le type de dosage utilisé et représentative de la limite de détection de la pathologie et
iii) établir le diagnostic.

La détermination de la quantité de transcrit majoritaire alternatif de *KLK8* et d'autre transcrit, le cas échéant alternatif, du même gène *KLK8* peut être mise en oeuvre séquentiellement ou simultanément, selon les procédés classiquement connus de l'homme du métier, comme indiqué précédemment.

Par même échantillon biologique, on entend un échantillon de même nature prélevé chez le même sujet, à savoir soit deux fractions d'un même prélèvement, soit deux échantillons issus de deux prélèvements différents mais qui doivent être de même nature, pas exemple du tissu cancéreux. On préfère utiliser deux fractions d'un même prélèvement.

Le procédé de diagnostic de l'invention peut également comprendre une étape supplémentaire de détection d'au moins un transcrit d'un gène d'une autre kallicréine, ce qui constitue un autre mode de réalisation de l'invention.

Le procédé ainsi détecte au moins un transcrit d'un gène d'une autre kallicréine, ainsi que :
(a) soit au moins un transcrit majoritaire alternatif du gène *KLK8* ou
(b) soit au moins un transcrit majoritaire alternatif du gène *KLK8* et au moins un transcrit minoritaire, le cas échéant alternatif, du gène *KLK8*.

Ce procédé peut être mis en oeuvre par les étapes consistant à :
i) déterminer la quantité de transcrit majoritaire alternatif du gène *KLK8*, et éventuellement de l'autre transcrit, le cas échéant alternatif, du gène *KLK8*, dans l'échantillon biologique Q1,
ii) déterminer la quantité du transcrit de l'autre gène de kallicréine dans le même échantillon Q2,
iii) calculer le rapport Q1/Q2 ou Q2/Q1,
iv) comparer ledit rapport à une valeur-seuil prédéteiminée, choisie selon le type de dosage utilisé et représentative de la limite de détection de la pathologie et
v) établir le diagnostic.

A titre d'exemple d'un autre gène de kallicréine approprié aux fins de l'invention, on peut citer les gènes de kallicréines s'exprimant dans le poumon, telles que *KLK5*, *KLK6*, *KLK7, KLK10, KLK11*, *KLK13* et *KLK14*. Ces gènes de kallicréines ont été largement décrits dans la littérature de sorte qu'ils sont connus de l'homme du métier. Les gènes de kallicréines *KLK5*, *KLK11* et *KLK13* sont préférés, *KLK11* étant particulièrement préféré.

Comme indiqué précédemment, la détermination de la quantité des transcrits de différente nature peut être mise en oeuvre séquentiellement ou simultanément, selon les procédés classiquement connus de l'homme du métier tels que décrits précédemment, et, par même échantillon biologique, on entend un échantillon de même nature prélevé chez le même sujet.

Outre la détection du transcrit majoritaire alternatif du gène *KLK8*, le procédé de diagnostic de l'invention peut également comprendre l'étape de détection d'au moins un transcrit d'un autre gène s'exprimant dans le poumon, sous-entendu différent d'un gène codant pour une kallicréine.

A tire d'exemple d'autres gènes s'exprimant dans le poumon, on peut citer les gènes codant les cadhérines desmosomales, telles que desmocolline 2 ou Dsc2 et desmogléine 2 ou Dsg2

Le procédé qui comprend en outre l'étape de détection d'au moins un transcrit d'un autres gène s'exprimant dans le poumon peut être mis en oeuvre par les étapes consistant à :
i) déterminer la quantité de transcrit majoritaire alternatif du gène *KLK8* dans l'échantillon biologique Q1,
ii) déterminer la quantité du transcrit de l'autre gène s'exprimant dans le poumon dans le même échantillon biologique Q4,
iii) calculer le rapport Q1/Q4 ou Q2/Q4,
iv) comparer ledit rapport à une valeur-seuil prédéterminée, choisie selon le type de dosage utilisé et représentative de la limite de détection de la pathologie et
v) établir le diagnostic.

Comme indiqué précédemment, la détermination de la quantité des transcrits de différente nature peut être mise en oeuvre séquentiellement ou simultanément, selon les procédés classiquement connus de l'homme du métier tels que décrits précédemment, et, par même échantillon biologique, on entend un échantillon de même nature prélevé chez le même sujet.

Dans chaque mode de réalisation de l'invention, la dernière étape consiste à établir le diagnostic.

Par diagnostic, on entend tant le diagnostic au sens large du terme, à savoir tant le diagnostic précoce que le dépistage, le suivi thérapeutique, le pronostic et le diagnostic des rechutes.

Le type de diagnostic va dépendre de la nature de l'échantillon biologique dans lequel le procédé de l'invention est mis en oeuvre. Ainsi, les fluides biologiques seront préférentiellement utilisés dans le cadre du diagnostic précoce, du dépistage, du diagnostic des rechutes et éventuellement du suivi thérapeutique. S'agissant des échantillons solides, tels que le tissu cancéreux, les ganglions lymphatiques ou les métastases, le cancer est déjà avéré. Le procédé de l'invention va donc être utile dans le cadre du pronostic et éventuellement du suivi thérapeutique.

Le procédé de l'invention est particulièrement approprié dans le pronostic de survie des patients atteints de cancer broncho-pulmonaire. En effet, les Demanderesses ont démontré qu'un taux élevé de transcrit majoritaire alternatif du gène *KLK8*, en particulier de NT3 et NT4, est un facteur pronostic défavorable du cancer et notamment du CBNPC.

Ainsi, un autre objet consiste en l'utilisation du procédé de diagnostic de l'invention dans le pronostic de survie des patients atteints de cancer broncho-pulmonaire.

Comme précédemment, le pronostic est amélioré par association de l'une des étapes suivantes :
- étape de détection d'au moins un autres transcrit, le cas échéant alternatif, du gène *KLK8*,
- étape de détection d'au moins un transcrit d'un autre gène de kallicréine, de préférence *KLK5*, *KLK11* et *KLK13*, *KLK11* étant particulièrement préféré, éventuellement en association avec la détection d'au moins un autre transcrit alternatif du gène *KLK8*,
- étape de détection d'au moins un transcrit d'un autre gène s'exprimant dans le poumon.

Pour la mise en oeuvre du procédé de diagnostic de l'invention, l'invention a pour autre objet un kit de diagnostic comprenant les outils nécessaires à la détection des transcrits majoritaires alternatifs du gène *KLK8*.

A titre non limitatif d'outils nécessaires à la détection des transcrits majoritaires alternatif du gène *KLK8*, on peut citer les partenaires de liaison desdits transcrits majoritaires, tels que les sondes d'hybridation et de détection.

L'invention concerne également l'utilisation d'au moins un des transcrits majoritaires alternatifs du gène *KLK8* codant la kallicréine 8 dans la production d'un agent utilisé dans un procédé de détection dans le cadre du cancer broncho-pulmonaire, notamment du cancer bronchique non à petites cellules, le procédé étant **caractérisé en ce qu**'il consiste à mettre en contact ledit au moins un transcrit majoritaire alternatif du gène *KLK8* avec un échantillon biologique issu d'un patient suspecté d'être atteint dudit cancer broncho-pulmonaire.

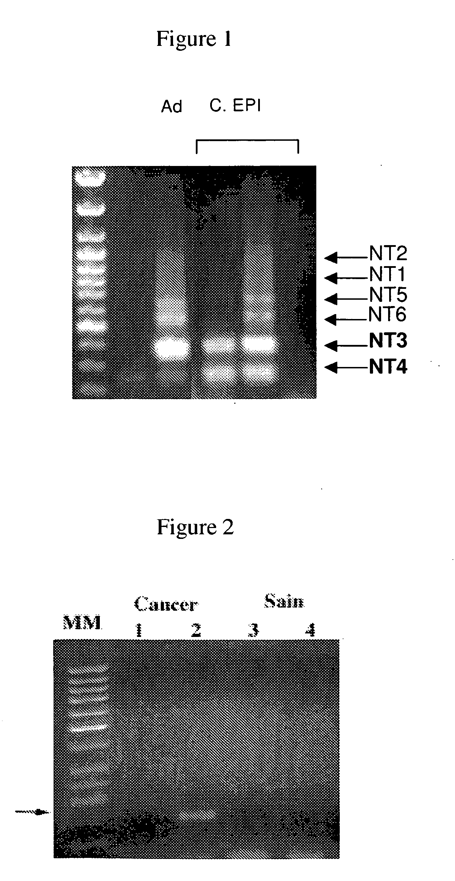

L'invention sera mieux comprise à l'aide des exemples suivants donnés à titre illustratif et non limitatif, ainsi qu'à l'aide des figures 1 et 2 annexées, sur lesquelles :
- la figure 1 est la photographie d'un gel d'électrophorèse montrant les différents transcrits alternatifs du gène *KLK8* NT1 à NT6 obtenus à partir de tissus cancéreux de patients atteints d'adénocarcinome (Ad) ou de carcinome épidermoïde (C. EPI), la piste de gauche correspondant à la piste des marqueurs de poids moléculaire et
- la figure 2 est la photographie d'un gel d'électrophorèse montrant la présence de transcrit NT4 dans le sang d'un patient atteint de carcinome, la piste de gauche correspondant à la piste des marqueurs de poids moléculaire (MM).

### Exemple 1 : Mise en évidence des transcrits pulmonaires majoritaires du gène KLK8 et des nouveaux transcrits

Des ARN totaux ont été extraits à partir de tissus cancéreux de patients atteints d'adénocarcinome ou de carcinome épidermoïde en utilisant le système «RNAeasy Midi kit » (Qiagen S.A., Courtaboeuf, France) selon les recommandations du fournisseur. Les ARN totaux ont été rétrotranscrits en ADNc à l'aide de la PowerScript Reverse Transcriptase (BD Biosciences Clontech, Palo Alto, CA).

Pour chaque échantillon, une réaction de transcription inverse a été effectuée à 42°C pendant une heure dans un volume final de 20 µl. Le milieu réactionnel était composé de 2 µg d'ARN totaux, 5 µM d'oligonucléotides décamériques aspécifiques (Random decamers RETROscript, Ambion, Cambridoeshire), de dNTP à la concentration chacun de 1 mM, de 20 U d'inhibiteur de RNase (Roche Diagnostics, Meylan), du tampon de réaction une fois concentré et d'une unité de Power Script Reverse Transcriptase (BD Biosciences Clontech, Palo Alto, CA).

Les ADNc ont été amplifiés par PCR. Le mélange réactionnel de 25 µl contenait : 50 ng d'ARN total rétro-transclit, 1 unité de FastStart Taq DNA (Polymérase Roche Diagnostics, Meylan), du tampon de réaction une fois concentré (50 mM Tris-HCl pH 8.3, 10 mM KCl, 5 mM (NH4)₂SO₄, 2 mM MgCl₂), des dNTP à la concentration de 0,3 mM et de 0,2 µM des amorces spécifiques.

Ces amorces ont été choisies de part et d'autre de la séquence codant la kallicréine KLK8 (NT1) et comportaient un site de coupure par une enzyme de restriction (*Not 1* ou *Eco*R V). Ces amorces étaient :
- K8Not_for : TGG AGG GCG GCC GCA TGG GAC GCC CCC GAC (SEQ ID N°1) et
- K8Eco_rev : TCC TAG ATA TCG CCC TTG CTG CCT ATG (SEQ ID N°2).
   Les réactions de PCR ont été réalisées dans un thermocycleur à gradient de température (Master cycler Gradient, Eppendorf). Les conditions d'amplification étaient les suivantes : un cycle de dénaturation de 5 min à 95 °C suivi de 45 cycles comportant une étape de dénaturation à 95°C pendant 20 s, une étape d'hybridation à 56°C pendant 20 s et une étape d'élongation à 72°C pendant 1,30 minutes. La réaction a été terminée par un cycle supplémentaire d'élongation de 1,30 min à 72°C.
   Dix microlites de la réaction ont été déposés sur un gel d'agarose à 0,8% contenant du bromure d'éthidium à 0,5 µg/ml. Les produits ont été séparés par électrophorèse et visualisés sous UV. Le marqueur de taille utilisé (Gene Ruler DNA Ladder mix) a été fourni par la firme MBI-Fermentas.
   La photographie du gel d'électrophorèse est donnée sur la figure 1 qui met en évidence les différents transcrits issus de l'expression pulmonaire du gène *KLK8* et la caractérisation de transcrits majoritaires des NT3 et NT4. Il est à noter que ces transcrits ont été identifiés par séquençage nucléotidique (voir exemple 2) comme étant les transcrits déjà décrits sous le vocable de Neuropsine de type 1 à 4 (NT1 à NT4), les deux nouveaux transcrits ayant été identifiés et dénommés NT5 et NT6.

### Exemple 2 : Caractérisation structurale des transcrits pulmonaires du gène KLK8 et conception d'amorces spécifiques.

Les produits de PCR obtenus dans l'exemple 1 ont été clonés dans le vecteur pcDNA5-FRT-V5-His TOPO (Invitrogen, Cergy Pontoise) selon les recommandations du fournisseur. La préparation d'ADN plasmidique a été réalisée à partir de différents clones en utilisant le système «Qiaprep MiniPrep» (Qiagen S.A, Courtaboeuf). L'ADN plasmidique purifié a ensuite été quantifié par spectrophotométrie à 260 nm, puis séquencé dans les 2 directions à l'aide du couple d'amorces T7 et pCR 3.1 (BGH_rev). Les séquences obtenues, données ci-après, ont été comparées aux séquences se trouvant dans les banques de données. La structure des transcrits clonés, donnée dans le tableau 1, a été déterminée par alignement avec la séquence du gène *KLK8* à l'aide du logiciel CLUSTAL W.
- *Séquence de l'ADNc pulmonaire YC170310.03 identique au transcrit NT1 (NM_007196). : SEQ ID N°3*
- *Séquence de l'ADNc pulmonaire YC140710.03 identique au transcrit NT2 (NM_144505).* : SEQ ID N°4
- *Séquence de l'ADNc pulmonaire YC090310.03 identique au transcrit NT3 (NM_144506)*. : SEQ ID N°5
- *Séquence de l'ADNc pulmonaire YC100310.03 identique au transcrit NT4 (NM_144507)*. : SEQ ID N°6
- *Séquence de l'ADNc pulmonaire YC210710.03 correspondant à un nouveau transcrit (NT5)*. : SEQ N°7
- *Séquence de l'ADNc pulmonaire YC050710.03 correspondant à un nouveau transcrit (NT6)*. : SEQ N°8

**Tableau 1**

| Transcrit pulmonaire | Nom générique | Structure |
|---|---|---|
| YC170310.03 | NT1 | EX1+EX2+EX3+E4+EX5+EX6 |
| YC140710.03 | NT2 | EX1+EX2+EX3 ALT+EX4+EX5+EX6 |
| YC090310.03 | NT3 | EX1+EX2+EX5+EX6 |
| YC100310.03 | NT4 | EX1+EX2+EX6 |
| YC210710.03 | NT5 | EX1+EX2+EX4+EX5+EX6 |
| YC050710.03 | NT6 | EX1+EX2+EX3+EX5+EX6 |

| | | |
|---|---|---|
| *EX = exon ; ALT*= *exon alternatif* | | |

Comme le montre le Tableau 1, les transcrits pulmonaires ne se distinguent les uns des autres que par des combinaisons différentes d'exons identiques. Il n'est donc pas possible de les cibler individuellement en jouant sur des séquences nouvelles (sauf NT2 qui possède une séquence additionnelle dans en 5' de l'exon 2). La connaissance du transcriptome pulmonaire du gène KLK8 permet de déterminer pour chaque transcrit la combinaison d'exons le distinguant des autres transcrits présents dans ce tissu, comme indiqué dans le Tableau 2.

**Tableau 2**

| Transcrit pulmonaire | Nom générique | Combinaison distinctive au niveau pulmonaire |
|---|---|---|
| YC170310.03 | NT1 | EX2-EX3+EX4-EX5 |
| YC140710.03 | NT2 | EX2-EX3ALT |
| YC090310.03 | NT3 | EX2-EX5 |
| YC100310.03 | NT4 | EX2-EX6 |
| YC210710.03 | NT5 | EX2-EX4 |
| YC050710.03 | NT6 | EX3-EX5 |

L'utilisation dans les sondes d'hybridation ou de détection des séquences jonctionnelles des exons présents dans ces combinaisons est donc le seul moyen de cibler spécifiquement et individuellement les transcrits pulmonaires. Ceci a été mis à profit pour générer l'amorce d'amplification conférant la spécificité de quantification des transcrits majoritaires NT3 et NT4 dans cet organe (voir Tableau 3).

**Tableau 3**

| Transcrit ciblé | Localisation de l'amorce | Nom de l'amorce spécifique sens et séquence (SEQ ID N°) |
|---|---|---|
| NT3 | Jonction EX2-EX5 | NT3_for : GGA GCC TGG GCA GAG AAT (SEQ ID N°9) |
| NT4 | Jonction EX2-EX6 | NT4 -2/6 : TGG GCA GGG CGA TTC T (SEQ ID N° 10) |

### Exemple 3: Quantification des transcrits NT3 et NT4 dans les tissus tumoraux de patients atteints de cancer pulmonaire.

Les transcrits NT3 et NT4 ont été dosés par PCR quantitative en temps réel en présence du fluorophore intercalant « SYBR Green » dans un thermocycleur iCycler iQ Détection Système (Biorad, Marnes la Coquette). Chaque essai incluait deux mesures de quantification des ADNc provenant d'échantillons pulmonaires tumoraux, deux mesures de témoins sans ADN et une courbe d'étalonnage construite en utilisant différentes dilutions d'ADN plasmidique étalon isolé des clones YC090310.03 (NT3) et YC100310.03 (NT4) (voir exemple 2). Les valeurs trouvées pour chaque échantillon ont été normalisées avec celle déterminée lors de la quantification des transcrits codant la sous-unité ribosomique 18S. Dans ce dernier cas, les courbes d'étalonnage ont été réalisées à partir de différentes dilutions d'une séquence de ce gène (853 pb) amplifiée par PCR conventionnelle et purifiée directement en utilisant le coffret Macherey Nagel selon les recommandations du fournisseur. Les oligonucléotides utilisés pour l'obtention de cette séquence étaient :
- l'oligo 18S_for : CTA CCA CAT CCA AGG AAG GCA GCA (SEQ ID N°11) et
- l'oligo 18S_rev : GCT ATC AAT CTG TCA ATC CTG TCC (SEQ ID N°12).

Le mélange réactionnel pour l'amplification quantitative de NT3 et NT4 comprenait : 100 ng d'ARN total rétro-transcrit (voir exemple 1), 1 unité de FastStart Taq DNA Polymérase, du SYBR Green (Roche Diagnostics, Meylan) 0,2 fois concentré, du tampon de réaction une fois concentré (50 mM Tris-HCl pH 8.3, 10 mM KCl, 5mM (NH4)₂S0₄, 2 mM MgCl₂), des dNTP à la concentration chacun de 0,2 mM et 0,2 µM de chaque amorce oligonucléotidique sens (Tableau III, exemple 2) et antisens du transcrit étudié. Les amorces antisens des transcrits NT3 et NT4 étaient respectivement : l'amorce NT3_rev (CCT CCA GAA TCG CCC T - SEQ ID N°13) hybridant avec la séquence de jonction des exons 5 et 6, et l'amome NT4_rev (CAG TCC AGG TAG CGG CAG - SEQ ID N°14) dont la séquence ciblée se trouve dans l'exon 6.

Les mesures de quantification du gène de ménage codant la sous-unité ribosomique 18S ont été effectuées dans le milieu réactionnel suivant : 0,5 ng d'ARN total rétro-transcrit, 1 unité de FastStart Taq DNA Polymérase, du SYBR Green 0,2 fois concentré, du tampon de réaction une fois concentré (50 mM Tris-HCl pH 8.3, 10 mM KCl, 5 mM (NH4)₂S0₄, 2 mM MgCl₂) auxquels ont été ajoutés 2 mM MgCl₂, des dNTP à la concentration chacun de 0,2 mM et 0,56 µM de chaque amorce sens (CGC GGT TCT ATT TTG TTG GTT TT - SEQ ID N° 15) et antisens (TTC GCT CTG GTC CGT CTT GC - SEQ ID N° 16).

Les conditions d'amplification pour la PCR quantitative des différents transcrits sont indiqués dans le Tableau 4.

**Tableau 4**

| Nombre de cycle(s) | Etape | Durée | Température | Transcrits |
|---|---|---|---|---|
| 1 cycle | Dénaturation | 5 min | 95 °C | NT3, NT4, 18S |
| | Dénaturation | 10 s | 95°C | NT3, NT4, 18S |
| 50 cycles | Hybridation | 10 s 10 s 10 s | 59 °C 61 °C 65 °C | NT3 NT4 18S |
| | Elongation | 15 s 20 s | 72 °C 72 °C | NT3, NT4 18S |
| | Acquisition de fluorescence | 15 s 15 s | 81 °C 84 °C | NT3, NT4 18S |

Les quantifications ont été réalisées à partir d'échantillons de tissus tumoraux prélevés sur les pièces d'exérèse chirurgicale de patients opérés d'un cancer broncho-pulmonaire entre janvier 2002 et juin 2004. La cohorte étudiée comportait 60 patients dont l'âge variait de 45 à 83 ans avec un âge médian égal à 65 ans (voir Tableau 5).

**Tableau 5**

| **Type tumoral** | **Nombre de patients** |
|---|---|
| Adénocarcinome | 33 |
| Carcinome épidermoïde | 16 |
| Carcinome à grandes cellules | 5 |
| Carcinome muco-épideimoïde | 1 |
| Carcinome neuro-endocrine | 1 |
| Tumeur carcinoïde | 4 |

Trente-huit patients avaient un cancer de stade 1 ou 2 selon la classification pTNM²⁴, et 24 patients, un cancer de stade 3 ou 4. L'analyse de la survie a été réalisée en collectant les informatisons relatives à leur état de santé en janvier 2006. Nous avons enregistré 26 décès dans la période de l'étude.

Pour les valeurs normalisées de NT3 et NT4 exprimées en unités arbitraires (UA), nous avons déterminé, à laide d'un test de χ2, une valeur seuil permettant de prédire au mieux la survie globale de la population. La valeur seuil est de 50 UA pour NT3 (χ2= 7,54 ; P=0,006) et de 1000 UA pour NT4 (χ2= 7,54 ; P=0,006). Ces valeurs, représentant les 45^{ème} et 46^{ème} percentiles, ont été utilisées pour les analyses ultérieures.

Les patients ont été classés en deux groupes : un groupe correspond aux individus présentant un niveau d'expression inférieur à la valeur seuil déterminée (Expression faible ; on parle de NT3 ou NT4 faible), et un groupe ayant un niveau d'expression supérieur (Expression forte ; on parle de NT3 ou NT3 faible). Pour chaque groupe, une courbe de survie a été construite selon la méthode de Kaplan-Meier et la significativité des différences entre les courbes a été évaluée par le *log rank test*. L'impact de l'expression des transcrits (comparaison expression forte versus expression faible) sur la survie globale des patients a été évalué par le HR (risque relatif de décès) qui a été calculé avec le modèle de Cox (Cox proportional hazards régression model). L'analyse a été réalisée de manière univariée. Elle a également été réalisée après ajustement avec le stade tumoral (ce qui permet d'éliminer la variable «stade ») puisque cette variable est fortement liée à la survie des patients (on parle alors de NT3 ou NT4 fort *ajusté*).

Les résultats sont indiqués dans le Tableau 6.

**Tableau 6**

| Variables | Kaplan-Meier et log rank test | Modèle de Cox | | |
|---|---|---|---|---|
| | *P* | HR | 95% IC | *P* |
| NT3 fort vs NT3 faible | 0,015 | 2,860 | 1,147 - 7,128 | 0,0241 |
| Stades 3+4 vs stades 1+2 | NC | 3,609 | 1,583-8,229 | 0,0023 |
| NT3 fort ajusté vs NT3 faible ajusté | | 2,300 | 0,911-5,804 | 0,0779 |
| NT4 fort vs NT4 faible | 0,005 | 3,608 | 1,448-8,994 | 0,0059 |
| Stades 3 +4 vs stades 1+2 | NC | 4,253 | 1,861-9,719 | 0,0006 |
| NT4 fort ajusté vs stades 1+2 | | 3,712 | 1,478-9,323 | 0,0052 |

| | | | | |
|---|---|---|---|---|
| *NC* : non calculé et *IC* : intervalle de confiance, vs = versus | | | | |

Les courbes de survie de Kaplan-Meier démontrent des différences significatives de survie (log rank test, Tableau 6) entre les patients exprimant fortement NT3 ou NT4 (NT3 ou NT4 fort) et les patients exprimant faiblement ces transcrits (P calculé par rapport à NT3 ou NT4 faible).

Le modèle de Cox permet de conclure que les patients exprimant fortement les transcrits ont une survie significativement inférieure aux autres patients. En effet, l'analyse de Cox montre que l'augmentation du risque de décès liée à une expression forte de NT3 (augmenté d'un facteur 2,860 ; HR, Tableau 6) ou de NT4 (augmenté d'un facteur 3,608 ; HR ,Tableau 6) est statistiquement significative. Les niveaux d'expression de ces transcrits constituent donc des indicateurs pronostiques de la survie des patients pour le cancer du poumon.

Après ajustement avec le stade tumoral (NT3 ou NT4 fort ajusté), on constate que l'augmentation du risque de décès lié à «NT3 fort » perd de la significativité statistique (P=0,0779). Ceci indique que les deux variables (expression de NT3 et stade tumoral) seraient liées dans cette étude.

Néanmoins, la forte tendance à la significativité de « NT3 fort ajusté » suggère que les deux variables pourraient se révéler indépendantes dans une étude plus robuste comportant un plus grand nombre d'évènements.

La situation est différente pour la variable « NT4 fort » puisque l'ajustement avec le stade tumoral ne modifie pas la significativité statistique de l'augmentation du risque de décès. Ce résultat prouve que la variable NT4 est un indicateur pronostique indépendant du stade tumoral.

### Exemple 4: Dosage des transcrits NT3 ou NT4 en association avec le dosage de transcrits d'autres gènes des kallicréines.

La stratégie de dosage des transcrits d'autres gènes des kallicréines s'exprimant dans le poumon (KLK5, *KLK6, KLK7, KLK10, KLK11, KLK13* et *KLK14*) est identique à celle décrite dans l'exemple 3. En résumé, la quantité de produits issus de l'amplification des ADNc et des contrôles a été déterminée après chaque cycle à l'aide de l'incorporation de SYBR green. Des courbes étalons ont été réalisées à partir d'ADN plasmidique provenant de clones d'ADNc des différents gènes. Les valeurs trouvées pour chaque patient et chaque gène ont été normalisées avec les valeurs du 18S ribosomique et exprimées en unité arbitraire. Les conditions réactionnelles étaient identiques à celles décrites pour NT3 et NT4 (Exemple 3). Les amorces de PCR utilisées sont décrites dans la Tableau 7.

**Tableau 7**

| Nom | Séquences | gènes | orientations | SEQ ID N° |
|---|---|---|---|---|
| K5.398_for | | *KLK5* | Sens | 17 |
| K5.682_rev | | | antisens | 18 |
| 256.K6_for | | *KLK6* | Sens | 19 |
| 393.K6_rev | | | antisens | 20 |
| 547.K7_for | | *KLK7* | Sens | 21 |
| 615.K7_rev | | | antisens | 22 |
| 2K10.210_for | | *KLK10* | Sens | 23 |
| 2K10.442_rev | | | antisens | 24 |
| K11_for2 | | *KLK11* | Sens | 25 |
| K11_rev2 | | | antisens | 26 |
| 576.K13_for | | *KLK13* | Sens | 27 |
| 672.K13_rev | | | antisens | 28 |
| 448.K14_for | | *KLK14* | Sens | 29 |
| 500.K14_rev | | | antisens | 30 |

**Tableau 8**

| Nombre de cycle(s) | Etape | Durée | Température | Transcrits |
|---|---|---|---|---|
| 1 cycle | Dénaturation | 5 min | 95 °C | tous |
| | Dénaturation | 10 s | 95°C | tous |
| 50 cycles | Hybridation | 15 s | 57 °C | KLK10 et 11 |
| | | 10 s | 59 °C | KLK14 |
| | | 10 s | 60 °C | KLK7 |
| | | 15 s | 65 °C | KLK5, 6 |
| | | 15 s | 68 °C | KLK13 |
| | Elongation | 15 s | 72 °C | KLK10 et 14 |
| | | 20 s | 72 °C | KLK5, 6, 7, 11 et 13 |
| | Acquisition de fluorescence | 15 s | 80 °C | KLK7 |
| | | 15 s | 81 °C | KLK 14 |
| | | 15 s | 84 °C | KLK6, 11 , 13 |
| | | 15 s | 86°C | KLK5, 10 |

Les cohortes utilisées pour le dosage des transcrits des différents gènes proviennent de la population étudiée dans l'exemple 3. La répartition des types histologiques tumoraux au sein des cohortes étudiées est donnée dans le Tableau 9.

**Tableau 9**

| **Type tumoral** | **KLK5, 7** | **KLK6** | **KLK10,11,13** | **KLK14** |
|---|---|---|---|---|
| Adénocarcinome | 29 | 33 | 28 | 33 |
| Carcinome épidermoïde | 16 | 16 | 16 | 15 |
| Carcinome à grandes cellules | 4 | 5 | 3 | 5 |
| Carcinome muco-épidermoïde | 1 | 1 | 1 | 1 |
| Carcinome neuro-endocrine | 1 | 1 | 1 | 1 |
| Tumeur carcinoïde | 3 | 4 | 1 | 4 |
| Nombre total | 54 | 60 | 50 | 59 |

Pour chaque patient, le niveau d'expression des différents transcrits a été déterminé puis a servi à calculer un rapport avec NT3 ou avec NT4 (Tableau 10 pour NT3 et Tableau 11 pour NT4). Pour chaque rapport, nous avons ensuite déterminé une valeur. seuil permettant de prédire au mieux la survie globale de la population à l'aide de la méthode décrite dans l'exemple 3.

**Tableau 10**

| **Variable** | **Seuil** | χ**2** | **P** | **percentile** | **décès** |
|---|---|---|---|---|---|
| NT3/KLK5 | 0,05 | 5,54 | 0,019 | 46 | 26 |
| NT3/KLK6 | 0,005 | 5,54 | 0,019 | 42 | 26 |
| NT3/KLK7 | 0,001 | 7,54 | 0,006 | 37 | 26 |
| NT3/KLK10 | 0,01 | 10,66 | 0,001 | 36 | 24 |
| NT3/KLK11 | 0,0015 | 8,17 | 0,004 | 40 | 24 |
| NT3/KLK 13 | 0,008 | 8,17 | 0,004 | 40 | 24 |
| NT3/KLK14 | 0,10 | 6,00 | 0,014 | 53 | 24 |

**Tableau 11**

| **Variable** | **Seuil** | χ**2** | **P** | **percentile** | **décès** |
|---|---|---|---|---|---|
| NT4/KLK5 | 2 | 3,84 | 0,049 | 53 | 26 |
| NT4/KLK6 | 2 | 3,84 | 0,049 | 43 | 26 |
| NT4/KLK7 | 0,1 | 7,50 | 0,006 | 39 | 26 |
| NT4/KLK10 | 0,4 | 5,99 | 0,014 | 42 | 24 |
| NT4/KLK11 | 0,1 | 10,66 | 0,001 | 44 | 24 |
| NT4/KLK13 | 0,7 | 8,17 | 0,004 | 46 | 24 |
| NT4/KLK14 | 0,5 | 5,99 | 0,014 | 52 | 24 |

Comme dans l'exemple 3, les patients ont été classés en deux groupes : un groupe correspond aux individus présentant un niveau d'expression inférieur à la valeur seuil déterminée (Expression faible), et un groupe ayant un niveau d'expression supérieur (Expression forte). Pour chaque groupe, une courbe de survie a été construite selon la méthode de Kaplan-Meier et la significativité des différences entre les courbes a été évaluée par le *log rank test*. Le risque relatif de décès (HR) a été calculé avec le modèle de Cox (Cox proportional hazards régression model). L'analyse a été réalisée après ajustement avec le stade tumoral puisque cette variable est fortement liée à la survie des patients.

Les résultats pour NT3 sont donnés dans le Tableau 12.

**Tableau 12**

| Variables | Kaplan-Meier et log rank test | Modèle de Cox | | |
|---|---|---|---|---|
| | *P* | HR | 95% IC | *P* |
| NT3/KLK5 fort vs NT3/KLK5 faible | 0,0214 | NC | NC | NC |
| Stades 3+4 vs stades 1+2 | NC | 3,549 | 1,553 - 8,110 | 0,0027 |
| NT3/KLK5 fort ajusté vs NT3/KLK5 faible ajusté | | 2,570 | 1,064 - 6,211 | 0,0360 |
| NT3/KLK6 fort vs NT3/KLK6 faible | 0,0378 | NC | NC | NC |
| Stades 3+4 vs stades 1+2 | NC | 3,662 | 1,609 - 8,335 | 0,0020 |
| NT3/KLK6 fort ajusté vs NT3/KLK6 faible | | 2,245 | 0,891 - 5,657 | 0,0864 |
| NT3/KLK7 fort vs NT3/KLK7 faible | 0,0564 | NC | NC | NC |
| Stades 3+4 vs stades 1+2 | NC | 3,572 | 1,564 - 8,158 | 0,0025 |
| NT3/KLK7 fort ajusté vs NT3/KLK7 faible ajusté | | 2,337 | 0,925 - 5,906 | 0,0727 |
| NT3/KLK10 fort vs NT3/KLK10 faible | 0,0126 | NC | NC | NC |
| Stades 3+4 vs stades 1+2 | NC | 3,476 | 1,517 - 7,966 | 0,0032 |
| NT3/KLK10 fort ajusté vs NT3/KLK10 faible | | 2,439 | 0,962-6,182 | 0,0602 |
| NT3/KLK11 fort vs NT3/KLK11 faible | 0,0085 | NC | NC | NC |
| Stades 3+4 vs stade 1+2 | NC | 3,958 | 1,743 - 8,990 | 0,0010 |
| NT3/KLK11 fort ajusté vs NT3/KLK11 faible ajusté | | 2,969 | 1,244 - 7,086 | 0,0142 |
| NT3/KLK13 fort vs NT3/KLK13 faible | 0,0041 | NC | NC | NC |
| Stades 3+4 vs stades 1+2 | NC | 3,473 | 1,520 - 7,935 | 0,0031 |
| NT3/KLK13 fort ajusté vs NT3/KLK13 faible ajusté | | 2,923 | 1,160 - 7,364 | 0,0229 |
| NT3/KLK14 fort vs NT3/KLK14 faible | 0,0205 | NC | NC | NC |
| Stades 3+4 vs stades 1+2 | NC | 3,669 | 1,604 - 8,392 | 0,0021 |
| NT3/KLK14 fort ajusté vs NT3/KLK14 faible ajusté | | 1,823 | 0,814 - 4,082 | 0,1444 |

| | | | | |
|---|---|---|---|---|
| NC : non calculé et IC : intervalle de confiance | | | | |

Cette étude établit que les patients ayant des rapports NT3/KLKx supérieurs à la valeur seuil présentent un taux de survie significativement diminué par rapport à ceux dont la valeur du rapport est inférieure à la valeur seuil (à l'exception de NT3/KLK7 ; log rank test).

Les résultats du test de Cox montre que les rapports NT3/ KLK5, NT3/KLK11, NT3/KLK13 sont des indicateurs pronostiques indépendants du stade tumoral (P< 0,05 pour la variable ajustée). La constitution d'un rapport de l'expression de NT3 sur l'expression des gènes KLK5, KLK11 et KLK13 améliore donc le pouvoir prédictif de NT3 puisque cette variable seule n'est pas totalement indépendante de la variable «stade tumoral » dans la population étudiée (voir exemple 3).

Les résultats pour NT4 sont donnés dans le Tableau 13.

**Tableau 13**

| Variables | Kaplan-Meier et log rank test | Modèle de Cox | | |
|---|---|---|---|---|
| | *P* | HR | 95% IC | *P* |
| NT4/KLK5 fort vs NT4/KLK5 faible | 0,0054 | NC | NC | NC |
| Stades 3+4 vs stade 1+2 | NC | 3,703 | 1,621 - 8,456 | 0,0019 |
| NT4/KLK5 fort ajusté vs NT4/KLK5 faible ajusté | | 3,131 | 1,349 - 7,270 | 0,0079 |
| NT4/KLK6 fort vs NT4/KLK6 faible | 0,0629 | NC | NC | NC |
| Stades 3+4 vs stade 1+2 | NC | 3,802 | 1,678 - 8,617 | 0,0014 |
| NT4/KLK6 fort ajusté vs NT4/KLK6 faible | | 2,073 | 0,864 - 4,974 | 0,1024 |
| NT4/KLK7 fort vs NT4/KLK7 faible | 0,0010 | NC | NC | NC |
| Stades 3+4 vs stades 1+2 | NC | 4,468 | 1,948 - 10,245 | 0,0004 |
| NT4/KLK7 fort ajusté vs NT4/KLK7 faible ajusté | | 3,657 | 1,458 - 9,178 | 0,0057 |
| NT4/KLK10 fort vs NT4/KLK10 faible | 0,0012 | NC | NC | NC |
| Stade 3+4 vs stades 1+2 | NC | 3,807 | 1,672 - 8,667 | 0,0014 |
| NT4/KLK10 fort ajusté vs NT4/KLK10 faible ajusté | | 3,802 | 1,521 - 9,503 | 0,0043 |
| NT4/KLK11 fort vs NT4/KLK11 faible | 0,0001 | NC | NC | NC |
| Stades 3+4 vs stades 1+2 | NC | 4,437 | 1,917 - 10,269 | 0,0005 |
| NT4/KLK11 fort ajusté vs NT4/KLK11 faible ajusté | | 6,362 | 2,330 - 17,366 | 0,0003 |
| NT4/KLK13 fort vs NT4/KLK13 faible | 0,0018 | NC | NC | NC |
| Stades 3+4 vs stade 1+2 | NC | 3,789 | 1,669-8606 | 0,0015 |
| NT4/KLK13 fort ajusté vs NT4/KLK13 faible ajusté | | 3,255 | 1,410 - 7,510 | 0,0057 |
| NT4/KLK14 fort vs NT4/KLK 14 faible | 0,0018 | NC | NC | NC |
| Stades 3+4 vs stades 1+2 | NC | 3,718 | 1,634 - 8,459 | 0,0017 |
| NT4/KLK14 fort ajusté vs NT4/KLK14 faible ajusté | | 2,821 | 1,217 - 6,537 | 0,0156 |

| | | | | |
|---|---|---|---|---|
| NC : non calculé et IC : intervalle de confiance | | | | |

Comme le montre le Tableau 13, les patients ayant des rapports NT4/KLKx supérieurs à la valeur seuil présentent un taux de survie significativement diminué par rapport à ceux dont la valeur du rapport est inférieure à la valeur seuil (à l'exception de NT3/KLK6 ; log rank test). Les résultats du test de Cox montre qu'hormis le rapport NT3/KLK6, tous les autres rapports constituent des indicateurs pronostiques indépendants du stade tumoral (P< 0,05 pour la variable ajustée). Dans cette étude, le rapport NT4/KLK11 apparaît particulièrement performant puisque le risque relatif de décès calculé avec cette variable est supérieur à celui obtenu avec le stade tumoral.

### Exemple 5 : Quantification du transcrit NT3 en association avec d'autres transcrits du gène KLK8 (NT1, NT2, NT5 et NT6), éventuellement en association avec d'autres transcrits de kallicréine

Dans les exemples précédents, les transcrits NT3 et NT4 ont été dosés séparément grâce à l'utilisation d'amorces de PCR discriminantes. Cet exemple vise à évaluer la valeur pronostique de ces transcrits lorsqu'ils sont dosés à l'aide d'amorces de PCR non discriminantes. Nous avons utilisé des oligonucléotides ciblant les exons 5 et 6 et permettant ainsi la quantification globale des transcrits NT1, NT2, NT3, NT5 et NT6. La variable mesurée a été dénommée « KLK8 ». Les séquences de ces amorces sont :
- 719.K8_for : CCA GAA GAA GTG TGA GGA TG (SEQ ID N°31) et
- 890.K8_rev : GGT ATA GAC GCC AGG TTT G (SEQ ID N°32).

Le mélange réactionnel utilisé était identique à celui de l'exemple 3 tandis que les condition d'amplification étaient : 1 cycle de 5 min à 95 °C puis 50 cycles comprenant une étape de dénaturation de 20s à 95 °C, une étape d'hybridation de 20 s à 60°C, une étape d'élongation de 20 s à 72°C et une étape d'acquisition de fluorescence de 15 s à 84°C. La procédure a été identique à celle des exemples précédents à savoir : (1) quantification de la variable à l'aide d'une courbe étalon, (2) normalisation avec le niveau d'expression de l'ARN ribosomique 18 S, (3) expression sous forme de valeur arbitraire et éventuellement mise en rapport avec l'expression d'un autre gène, (4) définition par test de Khi2 d'une valeur seuil (Tableau 14), (5) binérisation de la population (expression forte > seuil ; expression faible < seuil), (6) tests statistiques (Tableau 15).

Les cohortes étudiées étaient les mêmes que dans l'exemple 3 pour la variable « KLK8 », et que dans l'exemple 4 pour l'expression sous forme de rapport avec d'autres gènes de kallicréine.

**Tableau 14**

| **Variable** | **Seuil** | χ**2** | **P** | **percentile** | **décès** |
|---|---|---|---|---|---|
| KLK8 | 250 | 7,54 | 0,006 | 42 | 26 |
| KLK8/KLK5 | 0,33 | 5,53 | 0,018 | 43 | 26 |
| KLK8/KLK6 | 0,10 | 7,54 | 0,006 | 22 | 26 |
| KLK8/KLK7 | 0.020 | 15,39 | 0,00008 | 22 | 26 |
| KLK8/NT4 | 0,020 | 12,46 | 0,0004 | 19 | 26 |
| KLK8/KLK10 | 0,2 | 8,16 | 0,004 | 44 | 24 |
| KLK8/KLK11 | 0,02 | 8,16 | 0,004 | 42 | 24 |
| KLK8/KLK13 | 0,1 | 13,49 | 0,0002 | 40 | 24 |
| KLK8/KLK14 | 0,050 | 8,16 | 0,004 | 39 | 24 |

**Tableau 15**

| Variable | Modèle de Cox | | |
|---|---|---|---|
| | HR | 95% IC | *P* |
| Stades 3+4 vs stades 1+2 | 3,753 | 1,654 - 8,515 | 0,0016 |
| « KLK8 fort » ajustée vs KLK8 faible | 2,0152 | 0,856 - 5,412 | 0,1033 |
| Stades 3+4 vs stades 1+2 | 3,878 | 1,712 - 8,784 | 0,0012 |
| « KLK8/KLK5 fort » ajustée vs KLK8/KLK5 faible | 2,221 | 0,922 - 5,351 | 0,0752 |
| Stades 3+4 vs stades 1+2 | 4,129 | 1,830 - 9,132 | 0,0009 |
| « KLK8/KLK6 fort » ajustée vs KLK8/KLK6 faible | 0,752 | 0,299 - 1,889 | 0,5440 |
| Stade 3+4 vs stade 1+2 | 3,824 | 1,684 - 8,684 | 0,0013 |
| « KLK8/KLK7 fort » ajustée vs KLK8/KLK7 faible | 3,152 | 0,938 - 10,585 | 0,0633 |
| Stades 3+4 vs stades 1+2 | 4,152 | 1,833 - 9,401 | 0,0006 |
| « KLK8/NT4 fort » ajustée vs KLK8/NT4 | 0,851 | 0,290 - 2,501 | 0,7695 |
| Stades 3+4 vs stades 1+2 | 3,952 | 1,742 - 8,964 | 0,0010 |
| « KLK8/KLK10 fort » ajustée vs KLK8/KLK10 faible ajusté | 3,054 | 1,320 - 7,062 | 0,0091 |
| Stades 3+4 vs stades 1+2 | 3,725 | 1,639 - 8,470 | 0,0017 |
| « KLK8/KLK11 fort » ajustée vs KLK8/KLK11 faible | 3,247 | 1,352 - 7,797 | 0,0084 |
| Stades 3+4 vs stades 1+2 | 3,268 | 1,423 - 7,504 | 0,0052 |
| « KLK8/KLK13 fort » ajustée vs KLK8/KLK13 faible ajustés | 3,415 | 1,265 - 9,218 | 0,0153 |
| Stades 3+4 vs stades 1+2 | 3,249 | 1,487 - 7,911 | 0,0039 |
| « KLK8/KLK14 fort » ajustée vs KLK8/KLK14 faible ajustée | 2,178 | 0,852 - 5,573 | 0,1042 |

| | | | |
|---|---|---|---|
| IC, intervalle de confiance | | | |

Les patients ayant une forte valeur de la variable « KLK8 » ont une survie inférieure aux autres (log rank test, *P*= 0.0353) ; cependant cette variable est liée à la variable « stade tumoral » comme le montre le *P* non significatif de la variable ajustée (Tableau 17).

La variable « KLK8 » devient indépendante lorsqu'elle est combinée sous forme de rapport avec les niveaux d'expression d'autres gènes de kallicréine (P< 0,05 ; Tableau 17). C'est le cas des rapports KLK8/KLK10, KLK8/KLK11 et KLK8/KLK13. Ces variables constituent donc des indicateurs pronostiques défavorables et indépendants pour le cancer du poumon.

### Exemple 6: Mise en évidence de la présence du transcrit NT4 dans le sang de patients atteints d'un cancer du poumon.

Une détection sensible de carcinome latent à partir du sang périphérique de patients ayant un cancer pourrait avoir des implications pronostiques ou thérapeutiques importantes. Nous avons donc réalisé cette expérience afin de vérifier qu'il était possible de détecter la présence des transcrits NT4 dans le sang et que cette détection pouvait être associée à un cancer du poumon.

Du sang de sujets sains et de sujets atteints d'un cancer du poumon a été prélevé dans des tubes «PAXgene^{™} Blood RNA » (Europe BD) puis les ARN totaux ont été préparés à l'aide du PAXgene Blood RNA System (Qiagen, France) selon les préconisations des fournisseurs. Ces ARN totaux ont été rétro-transcrits en ADNc selon la procédure décrite dans l'exemple 1. La recherche du transcrit NT4 a été réalisée à l'aide d'une « Nested PCR » (2 PCR consécutives). Dans la première PCR, nous avons utilisé les amorces K8Not_for et K8Eco_rev décrites dans l'exemple 1. Le milieu réactionnel était identique à celui de cet exemple ainsi que les conditions de PCR. Toutefois, seuls 30 cycles d'amplification ont été réalisés. Pour la deuxième PCR, nous avons utilisé les amorces NT4-2/6 et NT4_rev décrites respectivement dans l'exemple 2 et 3. Le milieu réactionnel (contenant 1 µl de la première PCR) et le programme d'amplification étaient identiques à l'exemple 1, hormis le fait que la phase d'hybridation des amorces a été réalisée à 62°C. Cinquante cycles ont été effectués et 10µl du milieu de PCR ont été déposés sur un gel d'agarose coloré au bromure d'éthidium

Les résultats sont indiqués sur la figure 2 qui est une photographie du gel d'électrophorèse ainsi obtenu à partir de deux patients atteints de cancer et de deux patients sains.

Comme le montre la figure 2, il n'est pas possible de détecter le transcrit NT4 dans le sang de sujets sains. Cette observation indique l'absence de ce transcrit dans les cellules sanguines normales. Le transcrit NT4 a été détecté dans un des patients atteints d'un cancer du poumon. Cette approche permet donc de détecter la présence de cellules tumorales circulantes chez certains sujets ayant un cancer du poumon.

### Bibliographie

1 : Etzioni R. et al., 2003, Nature Reviews Cancer, 3 : 1-10
2 : Pisters KM et al, 2005, J Clin Oncol, 23:3270-3278,
3 : Bhattacharjee A. et al., 2001, Proc Natl Acad Sci USA, 98 : 13790-13795,
4: Garber M.E.et al., 2001, Proc Natl Acad Sci USA, 98 : 13784-13789,
5 : Planque C. et al., 2005, Biochemical and Biophysical Research Communications, 329 : 1260-1266,
6 : Dong Y. et al, 2001, Clin Cancer Res, 7 : 2363-2371,
7 : Dong Y. et al, 2003, Clin Cancer Res, 9 : 1710-1720,
8 : Yoshida S. et al, 1998, Gene, 213 : 9-16,
9 : Mitsui S. et al, 1999, Eur J Biochem, 260 : 627-634,
10 : Magklara A. et al, 2001, Clin Cancer Res, 7 : 806-811,
11 : P.E. Nielsen et al, 1991, Science, 254 : 1497-1500,
12 : Kricka et al., 1999, Clinical Chemistry, n° 45(4) : 453-458,
13 : Keller G.H. et al., 1993, DNA Probes, 2nd Ed., Stockton Press, sections 5 et 6, p.173-249,
14 : Tyagi & Kramer, 1996, Nature biotech, 14 :303-308
15 : M. Chee et al., 1996, Science, 274 : 610-614,
16 : A. Caviani Pease et al., 1994, Proc. Natl. Acad. Sci. USA, 91 : 5022-5026,
17 : G. Ramsay, 1998, Nature Biotechnology, 16 : 40-44,
18 : F. Ginot, 1997, Human Mutation, 10 : 1-10,
19 : J. Cheng et al, 1996, Molecular diagnosis, 1(3) : 183-200,
20 : T. Livache et al, 1994, Nucleic Acids Research, 22(15) : 2915-2921,
21 : J. Cheng et al, 1998, Nature Biotechnology, 16 : 541-546,
22 : Boom R. et al., 1990, J. Clin. Microbiol., 28(3) : 495-503,
23 : Levison PR et al., 1998, J. Chromatography, p. 337-344,
24 : Mountain, C.F., 1997, Chest, 111 : 1710-1717.

## Revendications

1. Procédé de diagnostic *in vitro* du cancer broncho-pulmonaire, notamment du cancer bronchique non à petites cellules, **caractérisé en ce qu'**il comprend l'étape de détection, dans un échantillon biologique issu d'un patient suspecté d'être atteint dudit cancer broncho-pulmonaire, d'au moins un des transcrits majoritaires alternatifs du gène *KLK8* codant la kallicréine 8.

2. Procédé de pronostic in vitro du cancer broncho-pulmonaire, notamment du cancer bronchique non à petites cellules, **caractérisé en ce qu'**il comprend l'étape de détection, dans un échantillon biologique issu d'un patient suspecté d'être atteint dudit cancer broncho-pulmonaire, d'au moins un des transcrits majoritaires alternatifs du gène KLK8 codant la kallicréine 8.

3. Procédé selon la revendication 1 ou 2, **caractérisé en ce que** les transcrits majoritaires alternatifs du gène 1 *KLK8* sont choisis parmi les transcrits NT3 et NT4.

4. Procédé selon la revendication 1 ou 2 ou 3, **caractérisé en ce qu'**il met en oeuvre les étapes consistant à :
i) déterminer la quantité de transcrit majoritaire alternatif dans l'échantillon biologique,
ii) comparer la quantité de transcrit majoritaire alternatif dans l'échantillon biologique à une valeur-seuil prédéterminée, choisie selon le type de dosage utilisé et représentative de la limite de détection de la pathologie et
iii) établir le diagnostic.

5. Procédé selon la revendication 1 ou 2 ou 3, **caractérisé en ce qu'**il comprend également l'étape de détection d'au moins un autre transcrit du gène *KLK8.*

6. Procédé selon la revendication 5, **caractérisé en ce que** le au moins un autre transcrit du gène *KLK8* est le transcrit codant la kallicréine KLK8 également appelé NT1.

7. Procédé selon la revendication 5, **caractérisé en ce que** le au moins un autre transcrit du gène *KLK8* est un transcrit alternatif.

8. Procédé selon la revendication 7, **caractérisé en ce que** le ou les transcrits alternatifs sont choisis parmi NT2, NT3, NT4, NT5 et NT6.

9. Procédé selon l'une quelconque des revendications 5 à 8, **caractérisé en ce qu'**il met en oeuvre les étapes consistant à :
i) déterminer la quantité de transcrit majoritaire alternatif du gène *KLK8* et la quantité de l'autre transcrit du gène *KLK8* dans le même échantillon biologique, et
ii) comparer la quantité obtenue à une valeur-seuil prédéterminée, choisie selon le type de dosage utilisé et représentative de la limite de détection de la pathologie et
iii) établir le diagnostic.

10. Procédé selon la revendication 1 ou 2 ou 5, **caractérisé en ce qu'**il comprend également l'étape de détection d'au moins un transcrit d'un gène codant une autre kallicréine.

11. Procédé selon la revendication 10, **caractérisé en ce qu'**il met en oeuvre les étapes consistant à :
i) déterminer la quantité de transcrit majoritaire alternatif du gène *KLK8* et éventuellement de l'autre transcrit du gène *KLK8* dans l'échantillon biologique Q1,
ii) déterminer la quantité du transcrit du gène codant l'autre kallicréine dans le même échantillon Q2,
iii) calculer le rapport Q1/Q2 ou Q2/Q1,
iv) comparer ledit rapport à une valeur-seuil prédéterminée, choisie selon le type de dosage utilisé et représentative de la limite de détection de la pathologie et
v) établir le diagnostic.

12. Procédé selon la revendication 1 ou 2 ou 3, **caractérisé en ce qu'**il comprend également l'étape de détection d'au moins un transcrit d'un autre gène s'exprimant dans le poumon non gène codant une kallicréine.

13. Procédé selon la revendication 12, **caractérisé en ce qu'**il met en oeuvre les étapes consistant à :
i) déterminer la quantité de transcrit majoritaire alternatif du gène *KLK8* dans l'échantillon biologique Q1,
ii) déterminer la quantité du transcrit de l'autre gène s'exprimant dans le poumon dans le même échantillon Q3,
iii) calculer le rapport Q1/Q3 ou Q3/Q1,
iv) comparer ledit rapport à une valeur-seuil prédéterminée, choisie selon le type de dosage utilisé et représentative de la limite de détection de la pathologie et
v) établir le diagnostic.

14. Utilisation d'un kit de diagnostic comprenant des partenaires de liaison des transcrits majoritaires alternatifs du gène *KLK8*, de préférence des sondes nucléiques ou des amorces d'amplification, pour la mise en oeuvre du procédé tel que décrit dans l'une quelconque des revendications 1, ou 3 à 12.

15. Transcrit NT5 alternatif du gène *KLK8* codant la kallicréine 8, **caractérisé en ce qu'**il est de séquence SEQ ID N°7.

16. Transcrit NT6 alternatif du gène *KLK8* codant la kallicréine 8, **caractérisé en ce qu'**il est de séquence SEQ ID N°8.

## Claims

1. A method for the *in vitro* diagnosis of bronchopulmonary carcinoma, in particular of non-small cell bronchial carcinoma, **characterized in that** it comprises the stage of detecting, in a biological sample derived from a patient suspected to be suffering from said bronchopulmonary carcinoma, at least one of the major alternative transcripts of the gene *KLK8* encoding kallikrein 8.

2. A method for the *in vitro* prognosis of bronchopulmonary carcinoma, in particular of non-small cell bronchial carcinoma, **characterized in that** it comprises the stage of detecting, in a biological sample derived from a patient suspected to be suffering from said bronchopulmonary carcinoma, at least one of the major alternative transcripts of the gene *KLK8* encoding kallikrein 8.

3. The method as claimed in claim 1 or 2, **characterized in that** the major alternative transcripts of the 1 *KLK8* gene are selected from the transcripts NT3 and NT4.

4. The method as claimed in claim 1 or 2 or 3, **characterized in that** stages consisting in:
i) determining the quantity of major alternative transcript in the biological sample,
ii) comparing the quantity of major alternative transcript in the biological sample with a predetermined threshold value, selected depending on the type of assay utilized and representative of the detection limit of the pathology and
iii) establishing the diagnosis, are implemented.

5. The method as claimed in claim 1 or 2 or 3, **characterized in that** it also comprises the stage of detecting at least one other transcript of the *KLK8* gene.

6. The method as claimed in claim 5, **characterized in that** the at least one other transcript of the *KLK8* gene is the transcript encoding kallikrein KLK8 also called NT1.

7. The method as claimed in claim 5, **characterized in that** the at least one other transcript of the *KLK8* gene is an alternative transcript.

8. The method as claimed in claim 7, **characterized in that** the alternative transcript or transcripts are selected from NT2, NT3, NT4, NT5 and NT6.

9. The method as claimed in any one of claims 5 to 8, **characterized in that** stages consisting in:
i) determining the quantity of major alternative transcript of the *KLK8* gene and the quantity of the other transcript of the gene *KLK8* in the same biological sample, and
ii) comparing the quantity obtained with a predetermined threshold value, selected depending on the type of assay utilized and representative of the detection limit of the pathology and
iii) establishing the diagnosis, are implemented.

10. The method as claimed in claim 1 or 2 or 5, **characterized in that** it also comprises the stage of detecting at least one transcript of a gene encoding another kallikrein.

11. The method as claimed in claim 10, **characterized in that** stages consisting in:
i) determining the quantity of major alternative transcript of the gene *KLK8* and possibly of the other transcript of the gene *KLK8* in the biological sample Q1,
ii) determining the quantity of the transcript of the gene encoding the other kallikrein in the same sample Q2,
iii) calculating the ratio Q1/Q2 or Q2/Q1,
iv) comparing said ratio with a predetermined threshold value, selected depending on the type of assay utilized and representative of the detection limit of the pathology and
v) establishing the diagnosis, are implemented.

12. The method as claimed in claim 1 or 2 or 3, **characterized in that** it also comprises the stage of detecting at least one transcript of another gene expressed in the lung which is not a gene encoding a kallikrein.

13. The method as claimed in claim 12, **characterized in that** stages consisting in:
i) determining the quantity of major alternative transcript of the *KLK8* gene in the biological sample Q1,
ii) determining the quantity of the transcript of the other gene expressed in the lung in the same sample Q3,
iii) calculating the ratio Q1/Q3 or Q3/Q1,
iv) comparing said ratio with a predetermined threshold value, selected depending on the type of assay utilized and representative of the detection limit of the pathology and
v) establishing the diagnosis, are implemented.

14. Utilization of a diagnostic kit containing binding partners of the major alternative transcripts of the *KLK8* gene, preferably nucleic acid probes or amplification primers for the implementation of the process as described in any one of claims 1 or 3 to 12.

15. An alternative transcript NT5 of the *KLK8* gene encoding kallikrein 8, **characterized in that** it has the sequence SEQ ID N°7.

16. An alternative transcript NT6 of the *KLK8* gene encoding kallikrein 8, **characterized in that** it has the sequence SEQ ID N°8.

## Patentansprüche

1. *In-vitro*-Diagnoseverfahren von bronchiopulmonalem Krebs, insbesondere von nicht-kleinzelligem Bronchialkrebs, **dadurch gekennzeichnet, dass** man in einem Schritt in einer biologischen Probe von einem Patienten, bei dem der Verdacht besteht, dass er an bronchiopulmonalem Krebs leidet, mindestens eines der hauptsächlichen alternativen Transkripte des *KLK8*-Gens, das für Kallikrein 8 codiert, nachweist.

2. *In-vitro*-Prognoseverfahren von bronchiopulmonalem Krebs, insbesondere von nicht-kleinzelligem Bronchialkrebs, **dadurch gekennzeichnet, dass** man in einem Schritt in einer biologischen Probe von einem Patienten, bei dem der Verdacht besteht, dass er an bronchiopulmonalem Krebs leidet, mindestens eines der hauptsächlichen alternativen Transkripte des *KLK8*-Gens, das für Kallikrein 8 codiert, nachweist.

3. Verfahren nach Anspruch 1 oder 2, **dadurch gekennzeichnet, dass** die hauptsächlichen alternativen Transkripte des Gens 1 *KLK8* aus den Transkripten NT3 und NT4 ausgewählt sind.

4. Verfahren nach Anspruch 1 oder 2 oder 3, **dadurch gekennzeichnet, dass** man folgende Schritte durchführt:
i) Bestimmen der Menge des hauptsächlichen alternativen Transkripts in der biologischen Probe,
ii) Vergleichen der Menge des hauptsächlichen alternativen Transkripts in der biologischen Probe mit einem zuvor festgelegten Schwellenwert, der je nach dem verwendeten Typ des Nachweises ausgewählt wird und für die Nachweisgrenze der Pathologie repräsentativ ist, und
iii) Festlegen der Diagnose.

5. Verfahren nach Anspruch 1 oder 2 oder 3, **dadurch gekennzeichnet, dass** man zudem in einem Schritt mindestens ein anderes Transkript des *KLK8*-Gens nachweist.

6. Verfahren nach Anspruch 5, **dadurch gekennzeichnet, dass** das mindestens eine andere Transkript des *KLK8-*Gens das auch als NT1 bezeichnete Transkript ist, das für Kallikrein KLK8 codiert.

7. Verfahren nach Anspruch 5, **dadurch gekennzeichnet, dass** das mindestens eine andere Transkript des *KLK8-*Gens ein alternatives Transkript ist.

8. Verfahren nach.Anspruch 7, **dadurch gekennzeichnet, dass** das oder die alternative(n) Transkript(e) aus NT2, NT3, NT4, NT5 und NT6 ausgewählt ist/sind.

9. Verfahren nach einem der Ansprüche 5 bis 8, **dadurch gekennzeichnet, dass** man folgende Schritte durchführt:
i) Bestimmen der Menge des hauptsächlichen alternativen Transkripts des *KLK8*-Gens und der Menge des anderen Transkripts des *KLK8*-Gens in derselben biologischen Probe und
ii) Vergleichen der erhaltenen Menge mit einem zuvor festgelegten Schwellenwert, der je nach dem verwendeten Typ des Nachweises ausgewählt wird und für die Nachweisgrenze der Pathologie repräsentativ ist, und
iii) Festlegen der Diagnose.

10. Verfahren nach Anspruch 1 oder 2 oder 5, **dadurch gekennzeichnet, dass** man zudem in einem Schritt mindestens ein Transkript eines Gens, das für ein anderes Kallikrein codiert, nachweist.

11. Verfahren nach Anspruch 10, **dadurch gekennzeichnet, dass** man folgende Schritte durchführt:
i) Bestimmen der Menge des hauptsächlichen alternativen Transkripts des *KLK8*-Gens und gegebenenfalls des anderen Transkripts des *KLK8*-Gens in der biologischen Probe Q1,
ii) Bestimmen der Menge des Transkripts des Gens, das für ein anderes Kallikrein codiert, in derselben Probe Q2,
iii) Berechnen des Verhältnisses Q1/Q2 oder Q2/Q1,
iv) Vergleichen des Verhältnisses mit einem zuvor festgelegten Schwellenwert, der je nach dem verwendeten Typ des Nachweises, ausgewählt wird und für die Nachweisgrenze der Pathologie repräsentativ ist, und
iii) Festlegen der Diagnose.

12. Verfahren nach Anspruch 1 oder 2 oder 3, **dadurch gekennzeichnet, dass** man in einem Schritt außerdem mindestens ein Transkript eines anderen Gens, das in der Lunge exprimiert wird und nicht für ein Kallikrein codiert, nachweist.

13. Verfahren nach Anspruch 12, **dadurch gekennzeichnet, dass** man folgende Schritte durchführt:
i) Bestimmen der Menge des hauptsächlichen alternativen Transkripts des *KLK8*-Gens in der biologischen Probe Q1,
ii) Bestimmen der Menge des Transkripts des anderen Gens, das in der Lunge exprimiert wird, in derselben Probe Q3,
iii) Berechnen des Verhältnisses Q1/Q3 oder Q3/Q1,
iv) Vergleichen des Verhältnisses mit einem zuvor festgelegten Schwellenwert, der je nach dem verwendeten Typ des Nachweises ausgewählt wird und für die Nachweisgrenze der Pathologie repräsentativ ist, und
v) Festlegen der Diagnose.

14. Verwendung eines Diagnosekits, das Bindungspartner der hauptsächlichen alternativen Transkripte des *KLK8-*Gens, vorzugsweise Nukleinsäuresonden oder Amplifikationsprimer, umfasst, für die Durchführung des Verfahrens nach einem der Ansprüche 1 oder 3 bis 12.

15. Alternatives Transkript NT5 des *KLK8*-Gens, das für Kallikrein 8 codiert, **dadurch gekennzeichnet, dass** es die Sequenz SEQ ID NR: 7 ist.

16. Alternatives. Transkript NT6 des *KLK8*-Gens, das für Kallikrein 8 codiert, **dadurch gekennzeichnet, dass** es die Sequenz SEQ ID NR: 8 ist.
